# EUROPÄISCHE PATENTSCHRIFT

(11) **EP 2 858 696 B1**
(45) Veröffentlichungstag und Bekanntmachung des Hinweises auf die Patenterteilung: **06.12.2017**
(21) Anmeldenummer: 13727053.4
(22) Anmeldetag: 06.06.2013
(51) Int. Cl.: A61M 1/36, B01D 61/24, A61K 35/00, A61K 35/16, C12N 5/0787

(54) **VERFAHREN ZUM HERSTELLEN EINER LEUKOZYTENPRÄPARATION UND LEUKOZYTENPRÄPARATION**
METHOD FOR PRODUCING A LEUKOCYTE PREPARATION, AND LEUKOCYTE PREPARATION
PROCÉDÉ PERMETTANT DE FABRIQUER UNE PRÉPARATION DE LEUCOCYTES, ET PRÉPARATION DE LEUCOCYTES

(30) Priorität: 08.06.2012 DE 102012209673
(43) Veröffentlichungstag der Anmeldung: 15.04.2015
(73) Patentinhaber: Artcline GmbH, 18057 Rostock (DE); Fraunhofer-Gesellschaft zur Förderung der angewandten Forschung e.V., 80686 München (DE)
(72) Erfinder: ALTRICHTER, Jens, 18196 Kavelstorf (DE); MITZNER, Steffen, 18119 Rostock (DE); LÜBCKE, Antje, 19053 Schwerin (DE); SELLENG, Kathleen, 17489 Greifswald (DE); DOSS, Fanny, 18059 Rostock (DE); KOCH, Stephanie, 18147 Rostock (DE)
(74) Vertreter: Seemann & Partner Patentanwälte mbB
(86) Internationale Anmeldenummer: PCT/EP2013/001664
(87) Internationale Veröffentlichungsnummer: WO 2013/182311

(56) Entgegenhaltungen:
- WO-A1-95/10291
- WO-A1-2005/032565
- US-A1- 2008 053 203
- MEYER ET AL: "Filter Buffy Coats (FBC): A source of peripheral blood leukocytes recovered from leukocyte depletion filters", JOURNAL OF IMMUNOLOGICAL METHODS, ELSEVIER SCIENCE PUBLISHERS B.V.,AMSTERDAM, NL, Bd. 307, Nr. 1-2, 20. Dezember 2005 (2005-12-20), Seiten 150-166, XP005221144, ISSN: 0022-1759, DOI: 10.1016/J.JIM.2005.10.004
- KAI HUBEL ET AL: "Effective storage of granulocytes collected by centrifugation leukapheresis from donors stimulated with granulocyte-colony-stimulating factor", TRANSFUSION, Bd. 45, Nr. 12, 1. Dezember 2005 (2005-12-01), Seiten 1876-1889, XP055070744, ISSN: 0041-1132, DOI: 10.1111/j.1537-2995.2005.00636.x

## Beschreibung

Die Erfindung betrifft ein Verfahren zum Herstellen einer Leukozytenpräparation aus einer Leukozytenfraktion. Die Erfindung betrifft weiter eine entsprechend hergestellte oder herstellbare Leukozytenpräparation sowie ihre Verwendung.

Die Transfusion von Leukozyten (weißen Blutkörperchen), insbesondere Granulozyten, ist eine unterstützende Therapie bei Patienten mit lebensbedrohlicher Neutropenie oder mit neutrophiler Dysfunktion. Granulozytentransfusionen können bei diesen Patienten auch Infektionen bekämpfen oder verhindern. Diese Granulozytentransfusionen bestehen aus Blutplasma mit angereicherten Granuloyzten, einer Leukozytenart, enthalten jedoch auch noch sehr hohe Zahlen an Thrombozyten (Blutplättchen) und Erythrozyten (roten Blutkörperchen). Für diese Anwendungen sind große Mengen von Granulozyten notwendig, üblicherweise von 1·10¹⁰ bis 8·10¹⁰ Granulozyten pro Apherese.

Bisher werden Granulozytenapheresate, auch "Granulozytenkonzentrate" genannt, aus dem Blut gesunder Spender gewonnen. In der Regel werden die Spender mit Steroiden, beispielsweise Glucocorticoiden wie Dexamethason oder Prednisilon, und/oder Wachstumsfaktoren, beispielsweise dem Granulozyten-Kolonie-stimulierenden Faktor ("Granulocyte colony-stimulating factor", G-CSF) 2 bis 24 Stunden vor der Spende stimuliert. Die Spende erfolgt durch Apherese, bei der das Blut über einen Katheter in einen Schlauch geführt wird, dort mit Zitrat und einem Sedimentationsbeschleuniger gemischt wird und in eine Zentrifuge überführt wird. In der Zentrifuge werden innen das Plasma und außen die Erythrozyten abgepumpt, gemischt und online dem Patienten rückinfundiert. Durch geeignete Pumpgeschwindigkeiten für Plasma und Erythrozyten wird die Leukozyten-haltige Zwischenschicht unter eine mittlere Auslassöffnung gebracht und von hier aus in einen Beutel gepumpt. Hierdurch werden "Granulozytenapheresate" gewonnen, die sehr unrein sind.

Die Begriffe "Granulozytenapheresat" oder "Granulozytenkonzentrat" täuschen, denn tatsächlich handelt es sich eher um ein Granulozyten-angereichertes Vollblut. Erythrozyten und Thrombozyten sind ca. 10-100fach mehr im Apheresat vorhanden als Leukozyten. Bei mit Steroid- und/oder G-CSF vorstimulierten Spendern sind in der Regel mehr als 50% der Leukozyten Granulozyten.

Solchermaßen gewonnenes Granulozytenapheresat ist nicht lagerfähig und muss je nach Landesgesetzen innerhalb von 6 Stunden bis maximal 24 Stunden klinisch eingesetzt werden. Dies liegt daran, dass Granulozyten im Blut nur eine kurze Lebensspanne haben, wobei die mittlere Blutverweilzeit ca. einen Tag beträgt. Des Weiteren werden Granulozyten und Monozyten durch unphysiologische Bedingungen schnell und sehr stark aktiviert, so dass Sauerstoffradikale und Enzyme freigesetzt werden, die die Zellen selbst auch schädigen ("Kamikaze-Effekt"). Diese kurze Lagerfähigkeit und die Aktivierung der Zellen stehen sowohl einer breiteren Untersuchung in der Forschung als auch der Anwendung zur Behandlung von Infektionen im Weg.

Um entsprechend große Mengen von Leukozyten, und insbesondere Granulozyten, zu erhalten, werden bislang Spender mit Glucocorticoiden und/oder so genanntem "Granulocyte colony-stimulating factor" (G-CSF) behandelt, was die Produktion von Granulozyten stimuliert, und nach dem Steigern der Granulozytenmenge im Spenderblut wird eine Apherese des peripheren Blutes ausgeführt, um diese Zellen zu sammeln. Die Stimulation des Spenders durch G-CSF kann mit negativen Effekten für den Spender wie Schmerzen oder Fieber assoziiert sein.

Eine alternative Möglichkeit, die ggf. ohne Stimulation des Spenders auskommt, ist die Herstellung einer Leukozytenpräparation aus Leukozytenfilmen ("buffy coats", abgekürzt BC) aus Vollblut ohne Apherese. Dazu werden die Vollblutspenden von ca. 450 ml bis 500 ml üblicherweise nach Sedimentation oder Zentrifugation fraktioniert in ein Erythrozytenkonzentrat und in Plasma. In dem Restbeutel, aus dem das Erythrozytenkonzentrat und das Plasma entfernt worden sind, verbleibt der Leukozytenfilm, der sich zwischen den beiden Fraktionen gebildet hat und der den überwiegenden Teil der Leukozyten und Thrombozyten des Spenders enthält. Erythrozyten sind in dem Leukozytenfilm abgereichert enthalten und dominieren den Leukozytenfilm noch immer.

In einigen Fällen werden die Leukozytenfilme mehrerer Vollblutspenden gepoolt, um Thrombozytenkonzentrate zu bilden. Dabei werden der größte Teil der Leukozytenfilme und insbesondere die Leukozyten als Abfall behandelt.

Die Verwendung von Leukozytenpräparationen ist an sich bekannt. Die Leukozyten sind aus verschiedenen Leukozytenarten aufgebaut, die verschiedene Eigenschaften aufweisen, unter anderem Granulozyten, Lymphozyten und Monozyten.

Für die klinische Einsetzbarkeit und die Einsetzbarkeit in der klinischen Forschung ist insbesondere die Lagerfähigkeit von Bedeutung. Einige häufig vorkommende Leukozytenarten wie die Granulozyten sind nur sehr kurz oder überhaupt nicht lagerbar und verlieren in bekannten Leukozytenpräparationen nach einigen Stunden bereits ihre Lebensfähigkeit und Funktionalität, so dass sie üblicherweise nicht länger als 24 Stunden gelagert und eingesetzt werden dürfen. Die üblicherweise eingesetzten Leukozyten- oder Granulozytenpräparationen sind außerdem stark mit Erythrozyten und Thrombozyten kontaminiert. Aufgrund der geringen Lagerbarkeit müssen solche Präparationen direkt nach der Blutspende oder der Apherese hergestellt und verwendet werden. Dies geschieht auf Anfrage.

Gemäß Bashir et al., Br. J. Haematol. 2008, 140 (6) 701-11, wurde eine Granulozytenpräparation aus Leukozytenfilmen hergestellt. Dazu wurden Leukozytenfilme gepoolt, mit 400 ml Thrombozytenadditivlösung gemixt und zentrifugiert. Die resultierende Granulozyten-angereicherte Schicht wurde unter Addition von 70 ml Plasma in einen Thrombozytenauffangbeutel transferiert. Das entsprechende Produkt enthielt durchschnittlich 8,8·10⁹ Neutrophile (Granulozyten). Die Thrombozytenkonzentration war nicht wesentlich reduziert. Die Granulozytenpräparation wurde 68 Stunden lang unter konstanten Bedingungen gelagert. Nach 68 Stunden war die Überlebensrate 84%, die Phagozytose und der respiratorische Burst im Vergleich zum Zeitpunkt unmittelbar nach der Herstellung auf 94% und 81% gesunken. Dabei tritt eine sehr starke Kontamination mit Erythrozyten und Thrombozyten auf.

In Mochizuki et al., Transfus. Med. 2007, 17 (4), 296 bis 303, wurde der Einfluss der Spendervorbehandlung auf die Granulozytenfunktion nach 72 Stunden mit Hilfe einer Beutelseparationsmethode untersucht. Eine unbehandelte Kontrolle, die einem Leukozytenfilm entsprach, wurde als Vergleich verwendet. Während der Lagerung bei Raumtemperatur wurde ein 10%iger Verlust von Granulozyten festgestellt. Die Überlebensrate (Viability) betrug 96%. Respiratorischer Burst und Phagozytose-Verhältnisse zum Anfang blieben verhältnismäßig stabil, wobei die Phagozytose gegenüber dem Anfang auf 83% gesunken war. Auch hierbei tritt eine sehr starke Kontamination mit Erythrozyten und Thrombozyten auf.

Demgegenüber liegt der vorliegenden Erfindung die Aufgabe zugrunde, ein Verfahren zum Herstellen einer Leukozytenpräparation, insbesondere einer Granulozytenpräparation, sowie eine entsprechende Leukozytenpräparation anzugeben, die über 24 Stunden hinaus lagerbar ist ohne wesentlichen Verlust an Lebensfähigkeit und Funktionalität der darin enthaltenen Leukozyten und insbesondere Granulozyten, wobei die Erythrozyten und Thrombozyten gegenüber den Leukozyten stärker als bislang möglich abgereichert sein sollen.

Diese Aufgabe wird durch Verfahren zum Herstellen einer Leukozytenpräparation aus einer Leukozytenfraktion mit den folgenden Verfahrensschritten gelöst:
a) Sedimentieren der Leukozytenfraktion, bis sich eine Trennlinie zwischen den sedimentierten Erythrozyten und einem Leukozyten-Überstand gebildet hat, Abtrennen des Leukozyten-Überstands vom Sediment, wobei der Leukozyten-Überstand in einem Leukozyten-Behälter aufgefangen wird oder verbleibt,
b) Sedimentieren der Leukozyten im Leukozyten-Behälter, wobei sich ein leukozytenarmer Überstand bildet, und Entfernen des leukozytenarmen Überstands aus dem Leukozyten-Behälter,
c) Waschen des Sediments im Leukozyten-Behälter mit einer Kochsalz enthaltenden Lösung, wobei die sedimentierten Zellen in der Kochsalz enthaltenden Lösung resuspendiert werden, die Lösung sedimentiert und ein resultierender Überstand entfernt wird, und
d) Resuspendieren des Sediments im Leukozyten-Behälter in einer Lagerlösung,
e) Lagern der Lagerlösung mit dem resuspendierten Sediment in einem gasdurchlässigen Beutel.

Im Sinne dieser Anmeldung werden unter dem Begriff Leukozytenfraktion solche Leukozyten enthaltende Zellgemische subsumiert, die als Leukozytenapheresat gewonnen wurden, ebenso aus Vollblut gewonnener Leukozytenfilm und andere, beispielsweise Kochenmarkpunktat, Stammzellapheresat, in Zellkultur behandelte Zellen, aber auch Vollblut selbst und Nabelschnurblut.

Dieses erfindungsgemäße Verfahren lässt sich sowohl bei Leukozytenapheresaten, aus Vollblutspenden stimulierter Spender als auch aus Leukozytenfilmen von Vollblutspenden nicht-stimulierter oder stimulierter Spender anwenden und beruht auf einer Kombination aus Sedimentationen und Waschungen. Die Leukozytenfraktion als Ausgangsmaterial enthält üblicherweise gerinnungshemmende Mittel, beispielsweise Citrat-Phosphat-Dextrose (CPD) oder Acid-Citrate-Dextrose (ACD). Die Verfahren zur Herstellung der Leukozytenfraktion als Leukozytenapheresat oder aus Leukozytenfilm ist an sich bekannt, letztere beispielsweise durch Zentrifugieren einer Vollblutspende bei hoher Beschleunigung, beispielsweise bei Raumtemperatur, Beschleunigung von 4000 g für 10 min.

Der Begriff "Leukozyten" im Zusammenhang mit "Leukozytenfraktion", "Leukozytenpräparation" o. ä. umfasst im Rahmen der Erfindung auch Granulozyten, Granulozytenfraktionen und Granulozytenpräparationen sowie Fraktionen anderer Leukozyten, die nicht Granulozyten sind.

Mit dem erfindungsgemäßen Verfahren lassen sich Leukozytenfraktionen gewinnen, bei denen im Vergleich zum Vollblut mehr als 90%, insbesondere mehr als 98% der Erythrozyten und mehr als 90% der Thrombozyten entfernt werden und die Zellen wenigstens 72 Stunden ohne wesentlichen Funktionsverlust lagerbar sind.

Der Verfahrensschritt e) der Lagerung erfolgt erfindungsgemäß in einem gasdurchlässigen Beutel. Auf diese Weise kann Kohlendioxid, das sich in der Lagerlösung bildet, entweichen bzw. entgasen, so dass der pH-Wert der Lösung weniger schnell absinkt als bei einem gasundurchlässigen Beutel, in dem sich Kohlensäure bildet, so dass die Lagerlösung versauert. Hierdurch wird die mögliche Lagerdauer verlängert.

Vorzugsweise wird die Lösung im Leukozyten-Behälter nach dem Verfahrensschritt d) in einem Verfahrensschritt e) gelagert, insbesondere bei einer Temperatur von -200°C bis +40°C, insbesondere bei +2°C bis +38°C, insbesondere bei +20°C bis +25°C, oder in einer Kryokonservierung bei -50°C bis -200°C eingefroren und gelagert. Damit können auch nach einer längeren Lagerdauer, beispielsweise bis zu 168 Stunden oder mehr, noch funktionierende Leukozytenpräparationen zur Verfügung gestellt werden.

Auch eine Kryokonservierung bei der Lagerung ist vorteilhaft, mit Einfrieren und Lagern bei -50°C bis -200°C, beispielsweise in flüssigem Stickstoff oder in einer Gasphase über flüssigem Stickstoff.

Vorzugsweise erfolgt in Verfahrensschritt a) das Sedimentieren bei einer Beschleunigung von 0,5 g bis 1000 g, insbesondere von 0,5 g bis 1,5 g oder von 50 g bis 1000 g, und/oder unter Zusatz eines Sedimentationsbeschleuniger, insbesondere Hydroxyethylstärke, Dextran, Ficoll, Percoll und/oder Gelatine, wobei insbesondere die Sedimentationsdauer zwischen 5 und 120 Minuten beträgt, vorzugsweise zwischen 20 und 45 Minuten. Das Sedimentieren der Leukozytenfraktion erfolgt somit bei geringer Beschleunigung, beispielsweise bei einer Beschleunigung von 1 g, also Erdbeschleunigung (Gravitation), oder wird durch Zentrifugation unterstützt. Hierbei kann ein Zusatz eines Sedimentationsbeschleunigers helfen. Das Verhältnis Zellgemisch zu Sedimentationsbeschleuniger beträgt vorzugsweise 1:0,2 bis 1:20, vorzugsweise 1:0,4 bis 1:2. Nach einigen Minuten trennt sich ein Sediment mit Erythrozyten von einem erythrozytenarmen Überstand. Der Überstand ist mit Leukozyten angereichert. Je nach Methode der Sedimentation wird entweder der Leukozytenüberstand in einen Leukozytenbehälter, beispielsweise einen Beutel, abgefüllt und somit vom Sediment getrennt oder das Sediment wird aus dem Leukozytenbehälter, in dem die Sedimentation stattgefunden hat, abgeführt und verworfen.

Das Sedimentieren der Leukozyten in Verfahrensschritt b) und oder das in Verfahrensschritt c) erfolgt vorzugsweise durch Zentrifugieren, insbesondere bei einer Beschleunigung von 50 bis 10000 g, insbesondere bei 100 bis 5500 g, insbesondere bei 200 bis 500 g. Auf diese Weise werden die Leukozyten von den Thrombozyten effektiv getrennt.

Die Sedimentation erfolgt vorzugsweise in einem flexiblen Beutel. Nach der Zugabe eines Sedimentationsbeschleunigers sollte vor der Sedimentation die vorhandene Luft vorteilhafterweise aus dem Beutel gepresst werden und ein Wiedereintritt von Luft durch Verschluss des Ausgangs verhindert werden (z.B. durch eine Klemme). Während der Sedimentation sollte der Beutel vorzugsweise nicht bewegt werden. Zur Luftverdrängung und unbewegten Lagerung wird vorteilhafterweise eine Vorrichtung genutzt, in die der Beutel eingehängt wird. Die Verdrängung des Sediments kann aus einem unteren Ausgang durch Druckdifferenz oder Schwerkraft, die des Überstandes aus einem oberen Ausgang durch Druckdifferenz erfolgen. Das Abziehen des Sediments durch Schwerkraft kann durch eine Klemme oder eine Pumpe gesteuert werden. Die Erzeugung einer Druckdifferenz zur Verdrängung des Sedimentes nach unten oder des Überstandes nach oben kann durch eine ansaugende Pumpe (Unterdruck) oder durch Erzeugung eines Überdruckes um den Sedimentationsbeutel herum unterstützt werden. Die Druckdifferenz kann manuell, beispielsweise durch eine manuelle Blutbeutelpresse, oder maschinell erzeugt werden. Beispielsweise kann der Sedimentationsbeutel vorteilhafterweise innerhalb eines weiteren Beutels oder einer druckdichten Kammer untergebracht sein, worin ein Überdruck durch Einleitung eines Gases oder einer Flüssigkeit erzeugt werden kann. Alternativ kann ebenfalls vorteilhafterweise im abziehenden Schlauch ein Unterdruck erzeugt werden, z.B. durch eine (Schlauch-)Pumpe. Das Ende der Trennung wird durch das Erreichen der jeweils anderen Komponente am Beutelausgang bestimmt und geht in der Regel einher mit einer Änderung der Farbe und/oder optischen Dichte des Inhaltes. Dieses Ereignis kann visuell durch den Durchführenden erkannt werden, in einem automatisierten System aber auch durch einen beispielsweise optischen Detektor.

Die Waschung in Verfahrensschritt c) wird vorzugsweise ein oder mehrere Male wiederholt. Die Wiederholung der Waschung dient der weiteren Aufreinigung der Leukozytenpräparation und Abreicherung von Thrombozyten und ggf. Erythrozyten.

Die Waschung oder Waschungen in Verfahrensschritt c) erfolgt oder erfolgen vorzugsweise mittels Zentrifugieren und/oder Filtration. Weiter vorteilhafterweise erfolgt oder erfolgen die Waschung oder Waschungen in Verfahrensschritt c) mit einer physiologischen Lösung, die insbesondere 50 bis 200 mM Natriumchlorid und/oder weitere Salze und/oder Puffer enthält.

Zur Überführung von Flüssigkeiten von einem Beutel in einen anderen während der Waschvorgänge und der Zugabe von Additiven können vorteilhaft die gleichen manuellen oder maschinellen Techniken benutzt oder die Schwerkraft genutzt werden, wie oben beschrieben zur Trennung von Sediment und Überstand.

Vorzugsweise ist die Lagerlösung ein blutgruppengleiches Blutplasma und/oder eine wässrige Lösung, die insbesondere blutgruppengleiches Plasma enthält.

Das erfindungsgemäße Verfahren wird vorzugsweise bei Raumtemperatur ausgeführt, insbesondere vorzugsweise bei 22 ± 2°C.

Vorzugsweise erfolgt das Verfahren in einem geschlossenen System steril, insbesondere in einem System von Schläuchen und Beuteln. Entsprechende Maßnahmen, Schläuche und Beutel steril miteinander zu verbinden und zu trennen, sind bekannt und werden standardmäßig angewandt. Hierzu werden beispielsweise entsprechende bekannte sterile Schlauchverschweißungen verwendet. Alternativ können vorteilhafterweise Schlauchverbindungen durch aseptische Techniken hergestellt werden, beispielsweise durch sterile Luer-Verbindungen oder geeignete sterile Dorne oder Kanülen in Konnektoren oder Durchstechsepten, wie sie bei Infusionssystemen Verwendung finden.

Vorzugsweise werden vor, während oder nach einem der Verfahrensschritte a) bis d) zur Verbesserung der Lagerung Additive zugegeben, insbesondere aus den Gruppen der pH-stabilisierenden Substanzen, insbesondere Puffer, der Nährstoffe, insbesondere Glukose, der Funktionsverbesserer, insbesondere Zytokine und/oder Wachstumsfaktoren, der Blutplasma-Komponenten, insbesondere blutgruppengleiches Blutplasma und/oder Albumin, und/oder der Antioxidantien, insbesondere Ascorbinsäure, Glutathion, Cystein, oder Methionin. Die Additive können auch noch während der Lagerung hinzugegeben werden.

Eine bevorzugte Additivlösung ist beispielsweise PAGGS-M, die neben Natriumchlorid, Natriumdihydrogenphosphat-Dihydrat und Dinatriumhydrogenphosphat-Dihydrat auch Glukose-Monohydrat, Mannitol, Adenin und Guanosin enthält. Dieses Additiv ist bevorzugt während der Lagerung einzusetzen. Ein weiteres vorteilhaft einsetzbares Additiv, das bevorzugt während der Lagerung eingesetzt wird, ist das Immunpräparat Immuno-akut®, das neben den Vitaminen B6, B12, C, D und E, auch Folsäure, diverse Carotinoide und Spurenelemente enthält. Die einzelnen genannten Bestandteile von PAGGS-M und/oder Immuno-akut® können auch einzeln als Additiv zugefügt werden.

Vorteilhafterweise werden vor, während und/oder nach einem der Verfahrensschritte a) bis d) mehrere Präparate gepoolt. Dies führt zu einer Ausschüttung von Zytokinen durch die Leukozyten, die durch ihre positive Auswirkung auf die Lebensdauer der Granulozyten die Lagerfähigkeit verbessern.

Erstmalig wird hiermit der Effekt der sogenannten "gemischten Leukozytenreaktion" (auch "gemischte Lymphozytenreaktion", "mixed leukocyte reaction", "mixed lymphocyte reaction", abgekürzt MLR) zur Lagerungsverlängerung von Granulozyten benutzt. Bei der MLR werden zwei Populationen allogener Lymphozyten (Lymphozyten genetisch unterschiedlicher Individuen der gleichen Spezies) gemischt, woraus primär eine T-Zell-Aktivierung, Mediatorfreisetzung und ggf. Proliferation resultiert. Die Zelloberflächenantigene des Hauptgewebeverträglichkeitskomplexes ("major histocompatibility complex", MHC) stellen dabei wahrscheinlich die größten Stimuli dar.

Die freigesetzten Mediatoren, vor allem Zytokine und Wachstumsfaktoren, aktivieren in einer gepoolten Leukozytenpräparation auch die anderen Leukozyten, z.B. Granulozyten, und verbessern deren Lagerfähigkeit und Aktivität. Alternativ oder zusätzlich kann ein Poolen mehrerer Leukozytenpräparationen auch erst nach der Lagerung in den letzten Stunden oder sogar Minuten vor der Anwendung erfolgen, um die Mediatorfreisetzung unmittelbar zur Stimulation der Leukozyten in der Leukozytenpräparation oder im Patienten zu nutzen.

Ein Einsatz einer gepoolten Leukozytenpräparation kann auch darin bestehen, den nahezu oder vollständig zellfreien, jedoch mediatorhaltigen Überstand der Leukozytenpräparation in ein separates Behältnis zu überführen und zur Immunstimulation in einen Patienten zu infundieren. Hierbei können an anderer Stelle genannte Transfertechniken benutzt werden.

Vorteilhafterweise findet vor oder nach wenigstens einem der Verfahrensschritte a) bis e) oder unmittelbar vor Anwendung eine Bestrahlung, insbesondere mit γ-Strahlen, statt, insbesondere mit einer Dosis von wenigstens 25 Gy, insbesondere wenigstens 30 Gy. Gemäß den Querschnitts-Leitlinien (BÄK) zur Therapie mit Blutkomponenten und Plasmaderivaten, 4. Auflage 2008, herausgegeben vom Vorstand der Bundesärztekammer auf Empfehlung des Wissenschaftlichen Beirats, Seite 157, und Schroeder M. L., "Transfusion-associated graft-versus-host disease", Br J Haematol 117, 275-287 (2002), bewirkt die Bestrahlung mit einer mittleren Dosis von 30 Gy (an keiner Stelle des Produktes weniger als 25 Gy) eine sichere Inhibition der T-Zell-Proliferation, während die Funktion von Erythrozyten, Thrombozyten und Granulozyten nach Bestrahlung weitgehend unbeeinträchtigt bleibt.

Die der Erfindung zugrunde liegende Aufgabe wird auch durch eine Leukozytenpräparation, gelöst, hergestellt oder herstellbar in einem erfindungsgemäßen zuvor beschriebenen Verfahren. Die Leukozytenpräparation zeichnet sich dadurch aus, dass gegenüber Vollblut die Erythrozyten um mehr als 90%, insbesondere mehr als 98%, und die Thrombozyten um mehr als 90% reduziert sind, während die Leukozyten um nicht mehr als 60% reduziert sind. Ferner ist sie bei einer Temperatur zwischen 2 °C und 38 °C für eine Dauer von mehr als 24 Stunden bis 168 Stunden ohne wesentlichen Funktionsverlust lagerbar, wobei insbesondere der respiratorische Burst und/oder die Phagozytose auch nach 72 Stunden der Lagerung noch mehr als 90% der entsprechenden Werte der frischen Leukozytenpräparation beträgt oder betragen.

Da die Leukozytenpräparation nach dem erfindungsgemäßen Verfahren herstellbar oder hergestellt ist, weist sie die entsprechenden Vorteile, Eigenschaften und Merkmale wie vorstehend beschrieben auf.

Weiter wird die der Erfindung zugrundeliegende Aufgabe auch durch eine erfindungsgemäße, zuvor beschriebene, insbesondere gepoolten, Leukozytenpräparation, geeignet zur Verwendung zur Herstellung eines im Wesentlichen zellfreien Überstandes mit hoher Mediatorkonzentration und/oder zur Verwendung in einem extrakorporalen Kreislauf oder zur Infusion. gelöst. Mit letztgenannten Alternativen sind klinische Forschung oder klinische Behandlungen mit Leukozyten- oder Granulozytenpräparationen ohne vorherige Anfrage möglich, da die Präparationen zur Verfügung stehen, wenn sie gebraucht werden, ohne eigens frisch hergestellt werden zu müssen. Die Verwendung einer erfindungsgemäßen Granulozytenpräparation in einem extrakorporalen Kreislauf ist beispielsweise in einem Verfahren möglich, wie es in Altrichter et al., Critical Care, 2001, 15:R82 "Extracorporeal cell therapy of septic shock patients with donor granulocytes: a pilot study" beschrieben ist.

Die Verwendung einer gepoolten Leukozytenpräparation zur Herstellung eines im Wesentlichen zellfreien Überstandes mit hoher Mediatorkonzentration, der in ein separates Behältnis zu überführen ist, hat den Vorteil, dass der Überstand zur Immunstimulation in einen Patienten infundiert werden kann. Hierbei können zuvor genannte Transfertechniken benutzt werden.

Vorteilhaft ist ebenfalls eine Verwendung der zellfreien flüssigen Anteile einer erfindungsgemäßen Leukozytenpräparation zur Infusion als Immunstimulanz.

Vorteilhaft zur Herstellung der erfindungsgemäßen Leukozytenpräparation sind auch ein Präparationssystem aus Beuteln und Schläuchen, das zur Durchführung eines zuvor beschriebenen erfindungsgemäßen Verfahrens zum Herstellen einer Leukozytenpräparation ausgebildet und eingerichtet ist, sowie eine Präparationsvorrichtung, die zur automatisierten oder teilautomatisierten Durchführung des zuvor beschriebenen erfindungsgemäßen Verfahrens zur Herstellung einer zuvor beschriebenen erfindungsgemäßen Leukozytenpräparation, insbesondere unter Verwendung eines zuvor beschriebenen erfindungsgemäßen Präparationssystems.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Figuren beschrieben, wobei bezüglich aller im Text nicht näher erläuterten erfindungsgemäßen Einzelheiten ausdrücklich auf die Zeichnungen verwiesen wird. Es zeigen:
- Fig. 1: die Anzahlen verschiedener Zelltypen in verschiedenen Verfahrensschritten des erfindungsgemäßen Verfahrens,
- Fig. 2: die Anzahlen von Zelltypen während der Lagerung einer erfindungsgemäßen Granulozytenpräparation,
- Fig. 3: die zeitliche Entwicklung der Phagozytose während der Lagerung,
- Figuren 4 bis 6: die zeitliche Entwicklung des respiratorischen Burst einer erfindungsgemäßen Granulozytenpräparation unter verschiedenen Bedingungen und Messmethoden während der Lagerung und
- Fig. 7 bis 13: Ergebnisse eines zweiten erfindungsgemäßen Ausführungsbeispiels,
- Fig. 14: Ergebnisse eines dritten erfindungsgemäßen Ausführungsbeispiels,
- Fig. 15 bis 18: Ergebnisse eines vierten erfindungsgemäßen Ausführungsbeispiels,
- Fig. 19 bis 24: Ergebnisse eines fünften erfindungsgemäßen Ausführungsbeispiels,
- Fig. 25 bis 31: Ergebnisse eines sechsten erfindungsgemäßen Ausführungsbeispiels,
- Fig. 32 bis 33: Ergebnisse eines siebten erfindungsgemäßen Ausführungsbeispiels.

### Erstes Ausführungsbeispiel

Im Folgenden wird an einem ersten Beispiel die erfindungsgemäße Herstellung einer Leukozytenpräparation bzw. Granulozytenpräparation aus Leukozytenfilmen (buffy coats) dargestellt.

### Herstellung der Leukozvtenfraktion

Zunächst wurden Vollblutspenden zwischen 450 ml bis 500 ml mit CPD bei 4000 g bei 22°C 10 min. lang zentrifugiert. Plasma, Leukozytenfilm und Erythrozyten wurden durch einen Plasmaexpressor (MacoPharma, Langen, Deutschland) innerhalb von vier Stunden nach der Spende separiert.

Zur Herstellung eines Leukozytenkonzentrats wurden die Leukozytenfilme verschiedener Spenden zusammengeführt (gepoolt). Alle Beutel- und Schlauchverbindungen wurden mit steriler Technik entsprechend Industriestandard durchgeführt.

Sechs Leukozytenfilme zwischen 62 ml und 82 ml von blutgruppenidentischen Spendern wurden in einer Reihe aneinander gefügt, d.h. die entsprechenden Beutel wurden in einer Reihe miteinander verbunden. Die Beutel wurden sanft massiert, um die an der Beutelinnenseite anhaftenden Leukozyten abzulösen. Der ausgiebig gemischte Inhalt dieser Leukozytenschichten wurde im letzten Beutel der Reihe zusammen aufgefangen (Pool-Beutel). Die Klammern zwischen den Beuteln der Reihe wurden geschlossen, um Reflux zu verhindern.

500 ml Hydroxyethylstärke (abgekürzt HES, Infukoll HES 6% 200/0.5 KS (Kochsalzlösung); Serumwerk, Bernburg, Deutschland) wurden in einen Transferbeutel gefüllt. Der Transferbeutel wurde unter stabilen Bedingungen an den ersten leeren Beutel der Reihe angeschweißt und 50 ml der HES-Lösung in diese Beutel transferiert, der damit ausgiebig durch Schwenken und manuelle Massage gespült wurde.

Dieser Verfahrensschritt wurde mit den folgenden Leukozytenschichtbeuteln wiederholt, indem jeweils die Schlauchpassage geöffnet wurde und der nächste Beutel mit 50 ml HES-Zellen-Suspension gefüllt wurde. Die Verbindung wurde wieder geschlossen, um Reflux zu verhindern. Schließlich wurde die HES-Lösung in den Pool-Beutel eingefüllt. Dieses "Auswaschen der Leukozytenfilmbeutel" wurde ein zweites Mal ausgeführt.

Anschließend wurde der Poolbeutel von der Leukozytenfilm-Beutelreihe abgetrennt und direkt an den HES-Beutel bzw. Transfer-Beutel angeschlossen. Der Pool-Beutel wurde vollständig mit HES-Lösung gefüllt und alle Luftblasen entfernt. Auf diese Weise wurden im Pool-Beutel 416 ± 13 ml Leukozytenfilm mit etwa 258 ± 30 ml HES-Lösung gemischt. Auf diese Weise wurde die LeukozytenFraktion hergestellt.

### Herstellung der Leukozytenpräparation

Zur Herstellung der Leukozytenpräparation aus der Leukozytenfraktion wurde der Pool-Beutel zunächst mit einem Transfer-Beutel verbunden, wobei die Verbindung noch geschlossen blieb. Diese Einheit wurde mit dem Verbindungsschlauch oben aufgehängt und 35 Minuten ungestört bei 22 ± 2° hängen gelassen, so dass die Erythrozyten durch die Schwerkraft sedimentierten und sich eine scharfe Trennlinie zwischen den sedimentierten Erythrozyten und dem Leukozyten-angereicherten Überstand bildete.

Der Pool-Beutel wurde in einen Plasma-Expressor (Mediros, Rostock, Deutschland) eingesetzt, um den angereicherten Überstand in den leeren Transfer-Beutel ("Leuko-Beutel") zu transferieren, nachdem die Schlauchverbindung geöffnet wurde. Das Erythrozytensediment wurde verworfen (vgl. Verfahrensschritt a) des erfindungsgemäßen Verfahrens).

Der Leuko-Beutel wurde mit einem leeren Beutel, beispielsweise einem ausgewaschenen Leukozytenfilm-Beutel der Beutelreihe verbunden, wobei die Verbindung noch geschlossen blieb. Die Beutel wurden dann bei 200 g bei 22 ± 2°C 7 min. lang zentrifugiert, so dass sich ein Sediment von Leukozyten und ein Plasma-HES-Lösungs-Überstand bildete, der die meisten Thrombozyten enthielt. Mittels des Plasmaexpressors wurde dieser Überstand in den leeren Beutel transferiert, der abgetrennt und verworfen wurde (vgl. Verfahrensschritt b) des erfindungsgemäßen Verfahrens).

Anschließend wurden zwei Waschungen ausgeführt, um die Anzahl der Thrombozyten weiter zu reduzieren, die noch im Leukozytensediment enthalten waren. Hierzu wurde der Leuko-Beutel mit einem Beutel mit 0,9%iger Kochsalzlösung verbunden und etwa 30 ml Kochsalzlösung in den Leuko-Beutel transferiert, um das Zellsediment durch vorsichtiges Streichen der Hand über den Beutel zu resuspendieren, bevor der Leuko-Beutel mit der Kochsalzlösung zu einem Gesamtgewicht von 350 g aufgefüllt wurde. Anschließend wurde der Beutel zentrifugiert bei 200 g, 22 ± 2°C für 7 min., die Waschlösung in einen leeren Beutel gepresst und dieser verworfen (vgl. Verfahrensschritt c des erfindungsgemäßen Verfahrens). Dieser Waschvorgang wurde einmal wiederholt.

Schließlich wurden die Leukozyten in 200 ml blutgruppenidentischen Spenderplasmas eines der Leukozytenfilmspenders resuspendiert, um die Leukozytenpräparation zu erhalten (Verfahrensschritt d).

### Lagerung der Leukozytenpräparation

Das resultierende Leukozytenkonzentrat bzw. die Leukozytenpräparation wurde in einen gasdurchlässigen Thrombozytenaufbewahrungsbeutel transferiert und Gasblasen entfernt. Die Leukozytenkonzentrate wurden bei Raumtemperatur ohne Agitation gelagert, wobei die Beutel auf Grillrosten lagen, um vollen Kontakt der Beuteloberfläche mit der Luft zu garantieren.

Auch in den weiteren Ausführungsbeispielen erfolgte die Lagerung jeweils in gasdurchlässigen Thrombozytenaufbewahrungsbeuteln.

### Analyse

Nach 24, 48 und 72 Stunden wurden die Leukozytenpräparationen jeweils gemischt und, nachdem der erste Milliliter verworfen wurde, also der Inhalt des Schlauchendes, jeweils Proben von 2 ml zur Analyse entnommen.

Die Konzentrationen von Erythrozyten, Thrombozyten und Leukozyten wurden durch automatisches Zählen (Sysmex KX-21N, Sysmex Corporation, Norderstedt, Deutschland) gemessen. Blutabstriche wurden hergestellt zur manuellen Differentiation der Leukozytenpopulationen, insbesondere Monozyten, Lymphozyten und Granulozyten.

Die Lebensfähigkeit der Leukozyten wurde durch einen Trypanblau-Ausschlusstest festgestellt.

Zur funktionellen Analyse mittels Chemilumineszenz-Analyse und Durchflusszytometrie wurden die Leukozytenkonzentrationen durch Addition von entsprechendem Blutplasma auf 5·10⁶ Leukozyten pro ml angepasst.

Die Sauerstoffradikalproduktion wurde durch Chemilumineszenz untersucht. Hierzu wurden zur Feststellung des respiratorischen Bursts (auch "oxidative burst" oder "Oxyburst") die Leukozyten mit serum-opsonisierten Zymosan-Partikeln (Sigma-Aldrich, Deisenhofen, Deutschland) stimuliert. Zur Negativkontrolle wurde Phosphatgepufferte Salzlösung (PBS) hinzugefügt. Die Chemilumineszenz wurde in zwei verschiedenen Tests durch Lucigenin und Luminol (Sigma-Aldrich, Deisenhofen, Deutschland) verstärkt.

Für die Chemilumineszenz-Analyse wurden die Proben jeweils dreifach in 96-Loch-Titerplatten gefüllt und die Lumineszenz durch ein automatisiertes Chemiluminescence-Messgerät (RS Luminoskan Ascent, Thermo Fisher Scientific, Dreieich, Deutschland) gemessen, direkt nachdem Zymosan zugegeben worden war. 60 Minuten lang wurde alle 2 Minuten die Chemilumineszenz in individuellen relativen Lichteinheiten (relative light units, RLU) bestimmt. Nach Abschluss der Messungen wurde das arithmetische Mittel der dreifachen Messungen berechnet und durch Aufsummieren dieser Mittel die "area-under-the-curve" (AUC) bestimmt.

Zur Messung der Funktionalität wurden die Phagozytose und respiratorischer Burst zusätzlich in Fluoreszenzzytometrie bestimmt. Hierzu wurde Heparin zu der Leukozytenpräparation in einer Konzentration von 25 internationalen Units (IU) per ml zugegeben. Zur Bestimmung der Phagozytose wurde das Testkit Phagotest® verwendet, zur Analyse des respiratorischen Burst das Testkit Phagoburst® (beide Orpegen, Heidelberg, Deutschland). Das Prinzip der Bestimmung der Phagozytose beruhte darauf, die Zellen mit obsonisierten inaktivierten Fluorescein-markierten ("FITC-labeled") E.coli bei einer Temperatur von 37°C 20 Minuten lang zu inkubieren. Negativkontroll-Proben wurden bei 4°C gelagert.

Der respiratorische Burst wurde durch Inkubieren der Zellen mit obsonisierten E.coli getestet. Die dabei resultierende Bildung von reaktiven Sauerstoffarten wurde 20 Minuten nach der Inkubation mit Dihydrorhodamin-123 (DHR-123) bei 37°C durch die Menge an fluoreszierendem Rhodamin bestimmt. Eine Probe ohne Zugabe von Bakterien diente als Negativ-Kontrolle.

Diese Reaktionen wurden gestoppt, indem kalte Quenching-Lösung, die Trypan-Blau umfasste, hinzugegeben wurde. Die Proben wurden zweifach mit kalter Salzlösung gewaschen. Erythrozyten wurden lysiert und Zellen fixiert, indem das dem Testkit beiliegende Lysis-Reagenz hinzugefügt wurde. Ein DNA-Anfärben wurde anschließend verwendet, um Artefakte, die durch Bakterienaggregate und Thrombozyten verursacht wurden, auszuschließen.

Die Proben wurden mittels FACS (fluorescence activated cell sorting), einer Durchflusszytometrie auf der Grundlage Fluoreszenzmarkierter Zellen, auf einem FACS Calibur (Becton Dickinson, San Jose, USA) innerhalb von 60 Minuten analysiert.

### Resultate

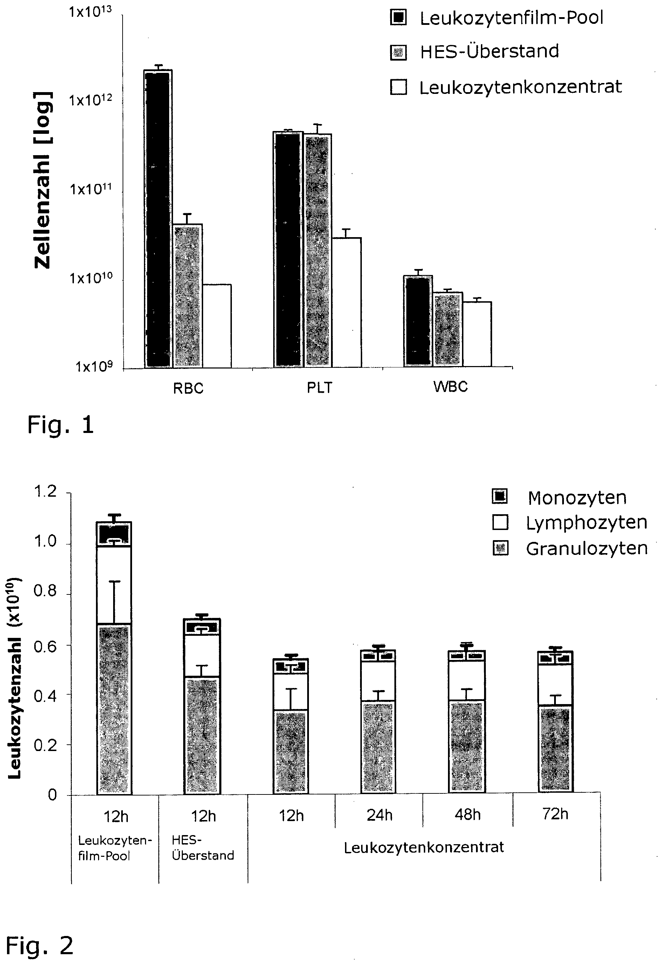

Die in den Figuren 1 bis 6 dargestellten Daten zeigen Mittelwerte als Balkenhöhe und Standardabweichungen als Fehlerbalken.

In Fig. 1 ist die Anzahl der Erythrozyten (RBC, "red blood cells"), Thrombozyten (PLT, "platelets") und Leukozyten (WBC, "white blood cells") für die Leukozytenfraktion (jeweils links), in dem HES-Überstand (jeweils Mitte) und in der finalen Leukozytenpräparation (jeweils rechts) durch Balkendiagramme dargestellt. Die Zellanzahl ist in einer logarithmischen Skala dargestellt. Es zeigt sich, dass die Anzahl der Erythrozyten gegenüber der Leukozytenfraktion bereits im HES-Überstand um mehr als 98% reduziert ist und in der Leukozytenpräparation nur noch 0,4% der ursprünglichen Menge enthalten ist. Die Konzentration der Thrombozyten ist im HES-Überstand lediglich um 7% reduziert, in der Leukozytenpräparation jedoch auf 6,1% der ursprünglichen Konzentration abgefallen. Die Konzentration der Leukozyten ist auf 49,8% der ursprünglich in der Leukozytenfraktion enthaltenen Konzentration abgefallen.

Damit sind die Leukozyten in der Leukozytenkonzentration wesentlich stärker gegenüber den Erythrozyten und den Thrombozyten aufkonzentriert als dies im Stand der Technik der Fall war.

In Fig. 2 sind die Anzahlen der Leukozytenarten im Leukozytenfilm-Pool (Leukozytenfraktion) nach 12 Stunden, im HES-Überstand nach 12 Stunden und in der endgültigen Leukozytenpräparation bzw. Leukozytenkonzentration nach 12 Stunden, 24 Stunden, 48 Stunden und 72 Stunden gezeigt, und zwar für die Leukozytenarten der Monozyten (oben), Lymphozyten (Mitte) und Granulozyten (unten).

Es zeigt sich, dass die Abnahme der Leukozytenkonzentration in den verschiedenen Verfahrensschritten die verschiedenen Arten der Leukozyten etwa gleichmäßig betrifft. Nach Herstellung der endgültigen Leukozytenpräparation ändern sich die Verhältnisse und die Anzahlen der verschiedenen Leukozytenarten in der Leukozytenpräparation nicht mehr wesentlich.

Die Überlebensrate ("vitality") betrug, wie in der folgenden Tabelle 1 dargestellt, mehr als 98% während der gesamten Beobachtungszeit.

**Tabelle 1**

| Zeit nach der Blutspende (h) | Vitalität (%) |
|---|---|
| 12 | 99.8 ± 0.3 |
| 24 | 99.7 ± 0.3 |
| 48 | 98.7 ± 0.3 |
| 72 | 98.7 ± 0.3 |

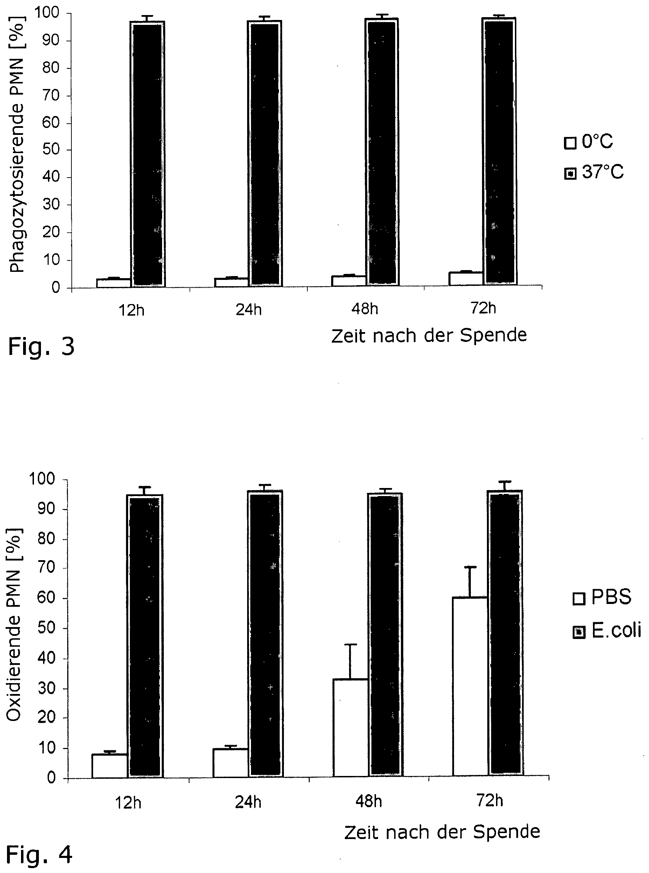

Wie in Fig. 3 dargestellt, betrug der Anteil der Granulozyten (PMN, "polymorphonuclear leucocytes"), die bezüglich Fluoresceinmarkierter E.coli Phagozytose ausübten, zu allen Zeitpunkten mehr als 95%, also während der gesamten Lagerungsdauer. Diese Aktivität wurde bei Körpertemperatur festgestellt, während bei 0°C die Aktivität stark reduziert ist. Die Messungen fanden mit Fluoreszenzzytometrie mit dem Testkit Phagotest® statt.

In Fig. 4 ist das Ergebnis der Messung der beim respiratorischen Burst der Granulozyten auftretenden Oxidation von DHR-123 in Reaktion auf die Präsenz von E.coli und in deren Abwesenheit gezeigt, gemessen in Fluoreszenzzytometrie mit dem Testkit Phagoburst®. Die mit E.coli versetzten Proben führten zu dem Ergebnis der rechten Balken zu den verschiedenen Zeitpunkten der Lagerung, die Negativkontrolle, die mit PBS versetzt war, führte zu den Aktivitäten gemäß den linken Balken. Hierbei zeigt sich, dass mehr als 95% der Granulozyten der Aktivprobe aktiv waren und eine Basisaktivität sich im Laufe der Lagerung steigerte.

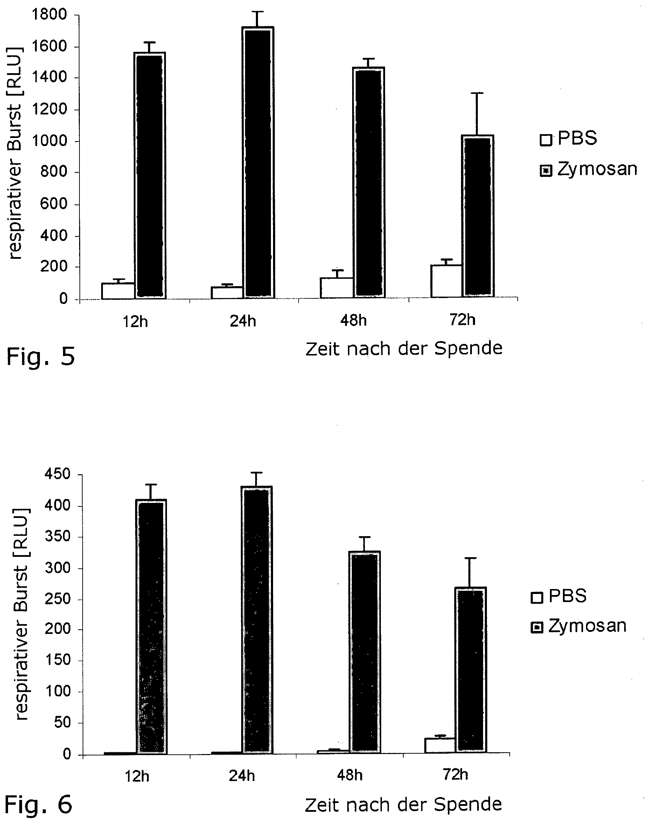

In Fig. 5 ist das Ergebnis einer Messung des oxidativen Burst der Proben der Leukozytenpräparation durch Messung der Chemilumineszenz mit Lucigenin gezeigt, wobei die Aktivprobe mit Zymosan versetzt wurde, die Negativprobe mit PBS. Die respiratorische Burst Antwort blieb in allen Fällen hoch, während sich eine leichte Basisaktivität zum Ende der Untersuchungsperiode hin aufbaut.

Das Gleiche ergibt sich gemäß Fig. 6 für die Untersuchung der Chemilumineszenz mit Luminol in der Leukozytenpräparation, ebenfalls in der Aktivprobe durch Zusatz von Zymosan und in der Passivprobe mit PBS.

### Zweites Ausführungsbeispiel

Das zweite Ausführungsbeispiel beschreibt die Herstellung der Leukozytenpräparation aus Granulozytenapheresaten. Die Analysen erfolgten analog zum ersten Ausführungsbeispiel.

Gesunde freiwillige Spender wurden entsprechend den gültigen deutschen Transfusionsbestimmungen mit Dexamethason und G-CSF einige Stunden vor der Apherese stimuliert, um die Granulozytenausbeute zu steigern. Die Apherese erfolgte entsprechend Herstellervorgaben auf einer COBE spectra (Caridian BCT, Garching Deutschland).

Unter sterilen Bedingungen wurde das Granulozytenapheresat direkt im Spendenbeutel mit Infukoll® HES 6% (Mw: 200.000) im Verhältnis 1:2 gemischt und anschließend zur Sedimentation mit den Verbindungsstücken nach oben aufgehängt. Während der Sedimentationsphase (ca. 40min bei 22 ± 2°C) wurde der Blutbeutel in regelmäßigen Abständen durch visuelle Kontrolle auf eine klare Trennschicht zwischen Erythrozytenpellet und leukozytenhaltigem Überstand überprüft. Sobald eine klare Trennschicht erreicht war (ca.40 Minuten), wurde der Überstand, der zum größten Teil Leukozyten und Thrombozyten enthielt, mit einer manuellen Blutpresse in einen neuen Beutel überführt. Um den Großteil der restlichen Thrombozyten zu entfernen, wurde der abgepresste Überstand zwei Mal mit physiologischer NaCI-Lösung (0,9%) gewaschen. Im Anschluss an den zweiten Waschschritt wurde das aufgereinigte Granulozytenkonzentrat in blutgruppenidentischem CPD-Plasma resuspendiert und in einem Thrombozytenlagerungsbeutel bei 22 ± 2°C unbewegt gelagert.

Durch die hier beschriebene Methode konnte eine Abreicherung von 98% der Erythrozyten und 95% der Thrombozyten erreicht werden. Während der Aufreinigung kam es lediglich zu einem Verlust von 26% der Leukozyten, so dass 74% der Leukozyten zur Lagerung zur Verfügung standen.

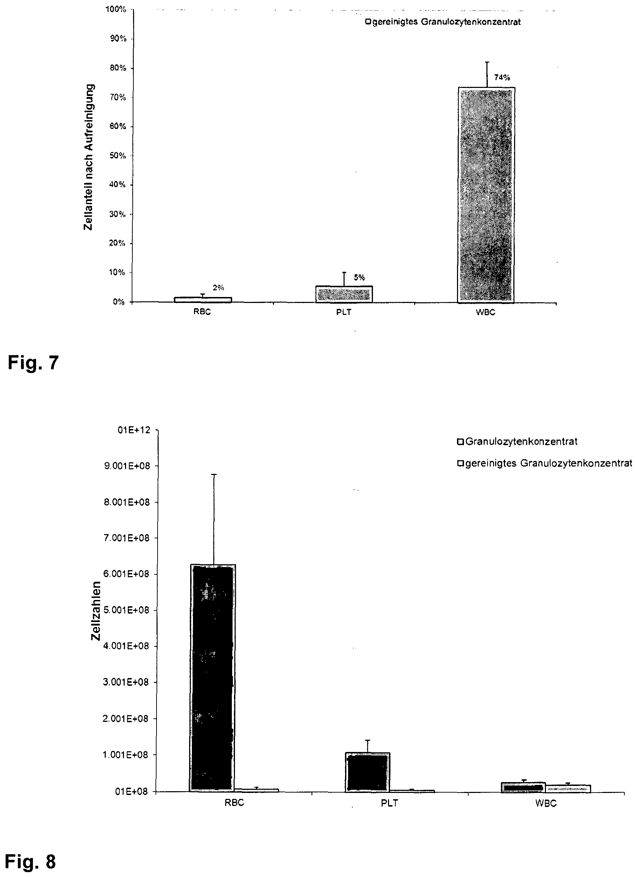

In den Figuren 7 bis 13 sind die Ergebnisse für die Leukozytenpräparation des zweiten Ausführungsbeispiels gezeigt.

So ist in Fig. 7 die Abreicherung der einzelnen Blutbestandteile Erythrozyten, Thrombozyten und Leukozyten zu erkennen, von denen 2%, 5% und 74% übrig geblieben sind.

In Fig. 8 sind die absoluten Zellzahlen dieser Blutbestanteile für das Ausgangspräparat, die Granulozytenfraktion bzw. das Granulozytenkonzentrat, und die erfindungsgemäße Granulozytenpräparation ("gereinigtes Granulozytenkonzentrat") gezeigt, in dem nunmehr die Leukozyten die Mehrheit bilden.

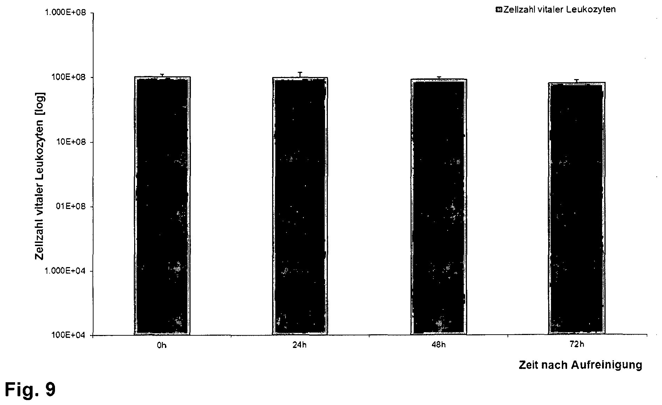

Aus Fig. 9 ist zu entnehmen, dass die Zellzahl vitaler Leukozyten in der Präparation auch nach 72 Stunden der Lagerung nicht wesentlich abgenommen hat.

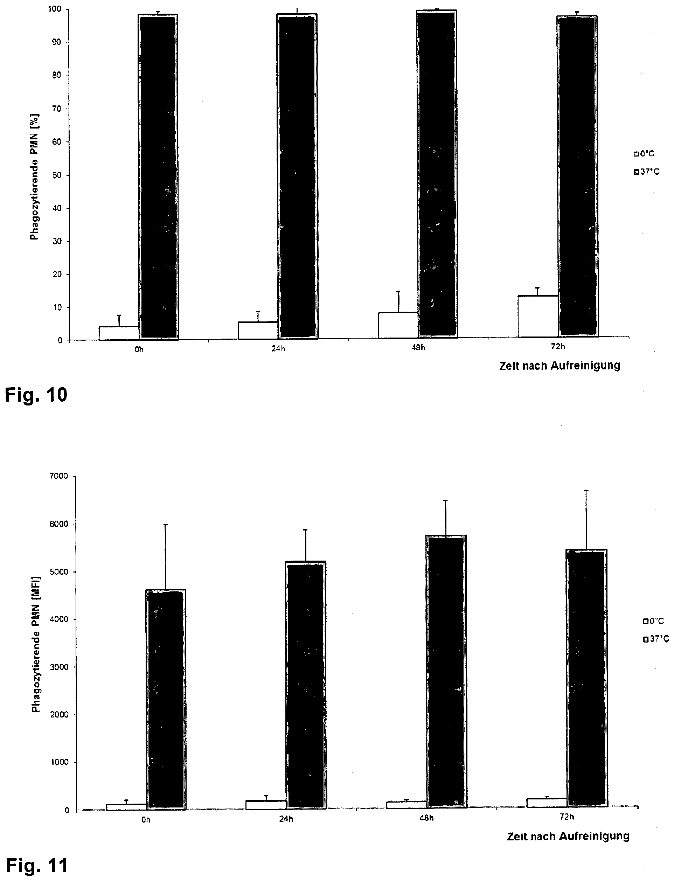

Fig. 10 zeigt, dass die relative Anzahl phagozytisierender Granulozyten im Laufe der Lagerung bis zu 72 Stunden nach der Aufreinigung bei fast 100% bleibt, während die Negativprobe nur leicht steigt. Fig. 11 zeigt hierzu den "mean fluorescence index" (MFI), der angibt, wie stark das durchschnittliche Fluoreszenz-Signal einer einzelnen Zelle ist. Dies ist bei 37°C gleichbleibend stark, die Negativprobe bleibt unauffällig.

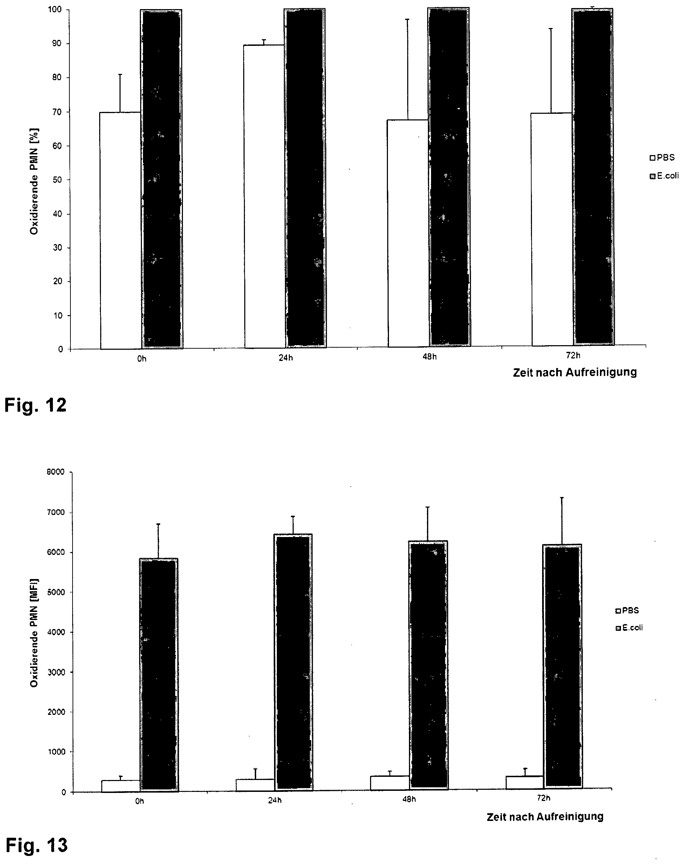

In Fig. 12 ist die relative Anzahl oxidierender Granulozyten gezeigt. Diese beträgt während der gesamten Lagerzeit nahe 100%, wobei auch die Granulozyten der Negativprobe zu einem großen Teil Oxidation betreiben. In Fig. 13 ist die mittlere Fluoreszenz-Antwort (ebenfalls "mean fluorescence index", MFI) pro Zelle in Bezug auf Fig. 12 dargestellt, woraus hervorgeht, dass die Aktivität der Granulozyten der Aktivprobe gleichbleibend stark ist, während die Negativprobe, wie zu erwarten, nur eine schwache Aktivität entfaltet.

Mit dem erfindungsgemäßen Verfahren und der erfindungsgemäßen Leukozytenpräparation sind die Nachteile der bisherigen Granulozytenapheresate bezüglich der mangelnden Reinheit, der kurzen Lagerfähigkeit und ggf. der Notwendigkeit einer Vorstimulation der Spender und deren Belastung durch Infusion eines Teils des Citrates und des Sedimentationsbeschleunigers bei Apherese beseitigt. Die erfindungsgemäße Leukozytenpräparation oder Granulozytenpräparation ist wenigstens 72 Stunden ohne wesentlichen Funktionsverlust lagerbar. Die Granulozyten- oder Leukozytenpräparation kann innerhalb einer kurzen Zeit, etwa dreieinhalb Stunden, und mit geringen Kosten hergestellt werden. Das Verfahren ist einfach durchführbar und kann in einem vollständig geschlossenen System durchgeführt werden, was der Sicherheit bezüglich potenzieller biologischer, toxikologischer oder chemischer Unreinheiten entgegenkommt. Ferner ist kein spezifischer Spender für die Präparation direkt notwendig, da die Leukozytenfilme in jeder Blutbank im normalen Betrieb in großen Mengen hergestellt werden. Es ist auch keine spezifische Spendervorstimulation mit Steroiden oder G-CSF notwendig, was die potenziellen Gesundheitsrisiken für Spender senkt und auch zu Kosten- und Zeitersparnis beiträgt.

### Drittes Ausführungsbeispiel

Das dritte Ausführungsbeispiel beschreibt die Herstellung einer Leukozytenpräparation aus Granulozytenapheresaten. Die Analysen erfolgten analog zum ersten Ausführungsbeispiel.

Die Herstellung der Granulozytenapheresate erfolgte analog zum zweiten Ausführungsbeispiel.

Unter sterilen Bedingungen wurde das Granulozytenkonzentrat direkt im Spendenbeutel mit HES 6% (Hydroxyethylstärke, Molekulargewicht: 200.000 Dalton) im Verhältnis 1:0,5 gemischt. Anschließend wurde ein Leerbeutel mit Hilfe eines Blutbeutel-Schlauch-Schweißgerätes (Terumo BCT Europe, Garching, Deutschland) steril angeschweißt, die Verbindungsnaht dabei aber nicht geöffnet und der Spendenbeutel mit dem Verbindungsschlauch nach oben für die Sedimentation aufgehängt. Während der Sedimentationsphase (ca. 40min bei Raumtemperatur) wurde der Spendenbeutel in regelmäßigen Abständen auf eine scharfe Trennschicht zwischen Erythrozytenpellet und leukozytenreichen Überstand visuell kontrolliert. Sobald eine solche Trennschicht vorhanden war, wurde die Naht im Verbindungsschlauch zwischen den Beuteln geöffnet und die leukozytenhaltige Phase (Überstand) mit einer manuellen Blutbeutelpresse in den vorhandenen Leerbeutel abgepresst.

In diesem dritten Ausführungsbeispiel wurden zwei unterschiedliche Varianten der weiteren Aufbereitung (Waschen des Leukozytensediments mit 0,9%iger NaCI-Lösung) verglichen.

In Variante 1 wurde nach dem Abpressen des leukozytenhaltigen Überstands ein neuer Leerbeutel an den Beutel mit dem leukozytenhaltigen Überstand steril angeschweißt (Verbindungsnaht blieb geschlossen) und der Überstand mit 300 g bei 22 ± 2°C 15 Minuten mit schwacher Bremse zentrifugiert. Nach dem Öffnen der Verbindungsnaht wurde der Überstand mit einer manuellen Blutpresse abgepresst und verworfen. Um den Großteil der Thrombozyten zu entfernen, wurde das Leukozytenpellet zwei Mal mit physiologischer NaCI-Lösung (0,9%) gewaschen und mit 300 g bei 22 ± 2°C 15 Minuten mit schwacher Bremse zentrifugiert (langsames Ausrollen über mehrere Minuten). Im Anschluss an den zweiten Waschschritt wurde das aufgereinigte Granulozytenkonzentrat in blutgruppenidentischem CPD-Plasma aufgenommen und analysiert.

Variante 2 des dritten Ausführungsbeispiels unterscheidet sich von Variante 1 des dritten Ausführungsbeispiels in den Kennzahlen der Zentrifugationsschritte. So wurden der Sedimentationsüberstand und die in 0,9%iger NaCI-Lösung resuspendierten Leukozytenpellets jeweils mit 300 g bei 4°C 5 Minuten mit starker Bremse zentrifugiert (vergleichsweise schneller Stopp innerhalb von weniger als einer Minute).

Durch die beiden hier beschriebenen Varianten der Aufreinigung konnte eine Abreicherung von mehr als 98% der Erythrozyten erreicht werden. Die beiden Varianten zeigen aber deutliche Unterschiede in der Abreicherung an Thrombozyten. So wurden bei der Variante 1 (15-minütige Zentrifugation mit schwacher Bremse) lediglich 70% der Thrombozyten abgereichert, wohingegen bei der Variante 2 (5-minütige Zentrifugation) 95% entfernt werden konnten. Bei Variante 1 kam es während der Aufreinigung lediglich zu einem Verlust von 27% der Leukozyten und bei Variante 2 von 33%.

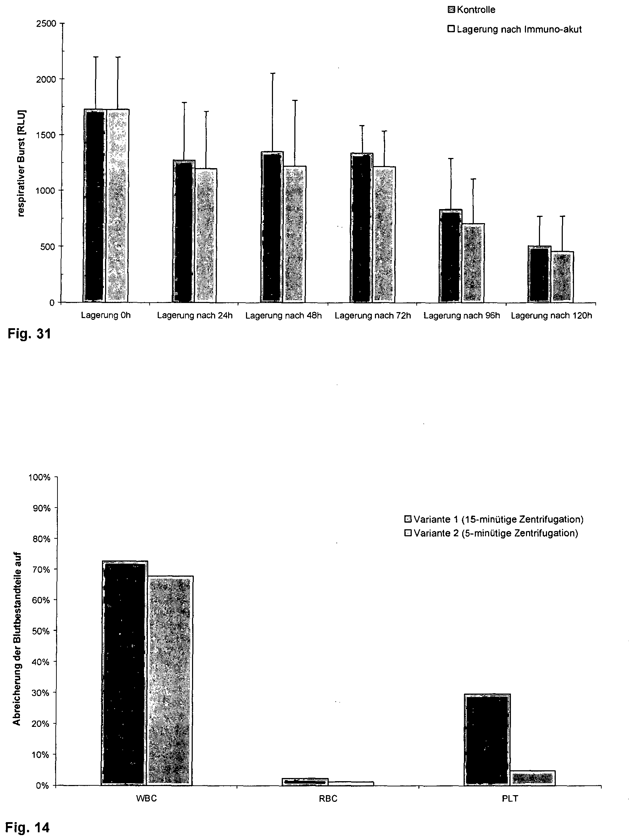

In Fig. 14 ist die Abreicherung der einzelnen Blutbestandteile Leukozyten (WBC, "white blood cells"), Erythrozyten (RBC, "red blood cells") und Thrombozyten (PLT, "platelets") beider Varianten zu erkennen, von denen bei Variante 1 73%, 2% und 30% und bei der Variante 2 67%, 1% und 5% erhalten geblieben sind (Mittelwerte aus je 2 Versuchen).

### Viertes Ausführungsbeispiel

Das vierte Ausführungsbeispiel beschreibt die Herstellung einer Leukozytenpräparation aus Granulozytenapheresaten nach der im dritten Ausführungsbeispiel beschriebenen Variante 2 und der anschließenden Lagerung in blutgruppenidentischem CPD-Plasma über einen Zeitraum von 72 Stunden mit Pufferung des pH-Werts.

Im Anschluss an die Sedimentation und Aufreinigung des Granulozytenapheresats wurde die Zellkonzentration auf 5•10⁷ Leukozyten/ml eingestellt, so dass 1•10¹⁰ Leukozyten in 200 ml CPD-Plasma in einem Thrombozytenlagerungsbeutel ohne Agitation bei Raumtemperatur gelagert wurden. Der Thrombozytenlagerungsbeutel lag dabei auf Gitterrosten, um vollen Kontakt der Beuteloberfläche mit der Luft zu gewährleisten. Die Lagerung wurde im Doppelansatz durchgeführt, wobei bei einem der Lagerungsansätze der pH-Wert, sobald er kleiner als pH 7,2 war, mit einer entsprechenden Menge Natriumbicarbonat-Lösung 8,4% (NaBic 8,4%) so korrigiert wurde, dass er sich wieder im physiologischen Bereich von humanem Blut (7,35 - 7,45) befand.

### Analyse

Nach 0, 24, 48, 72 und 144 Stunden wurden die beiden Leukozytenlagerungsansätze gut gemischt und jeweils Proben von 3 ml zur Analyse unter sterilen Bedingungen entnommen.

Zu jedem Zeitpunkt wurden die Zellkonzentration und die Gesamtzellzahl der Proben bestimmt. Weiterhin wurde die Vitalität mittels Trypanblau-Ausschlusstest ermittelt und die Funktionalität im Bereich Phagozytose und respiratorischer Burst mit der Fluoreszenzzytometrie untersucht. Die Durchführung aller Analysen wurde wie im ersten Ausführungsbeispiel beschrieben durchgeführt.

### Resultate

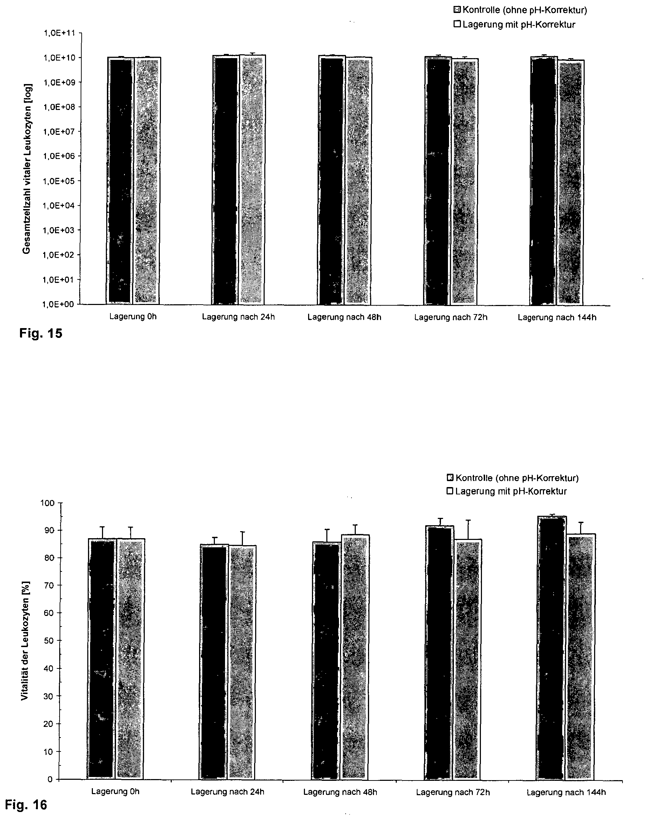

Die in den Figuren 15 bis 18 dargestellten Daten sind Mittelwerte und deren Standardabweichungen.

Aus Fig. 15 ist zu entnehmen, dass die Zellzahlen vitaler Leukozyten über den Lagerungszeitraum von 144 Stunden nicht wesentlich abnehmen. Außerdem ist festzustellen, dass die Pufferung des pH-Werts mit Natriumbicarbonat-Lösung 8,4% keine negativen Auswirkungen auf die Zellzahlen hat.

Auch die in Fig. 16 dargestellten Vitalitäten der Leukozyten über die Lagerungsdauer von 144 Stunden zeigen, dass es zu keiner negativen Beeinflussung durch die pH-Pufferung kommt. Auch am Ende der Lagerung liegt die Vitalität beider Lagerungsvarianten bei >85%.

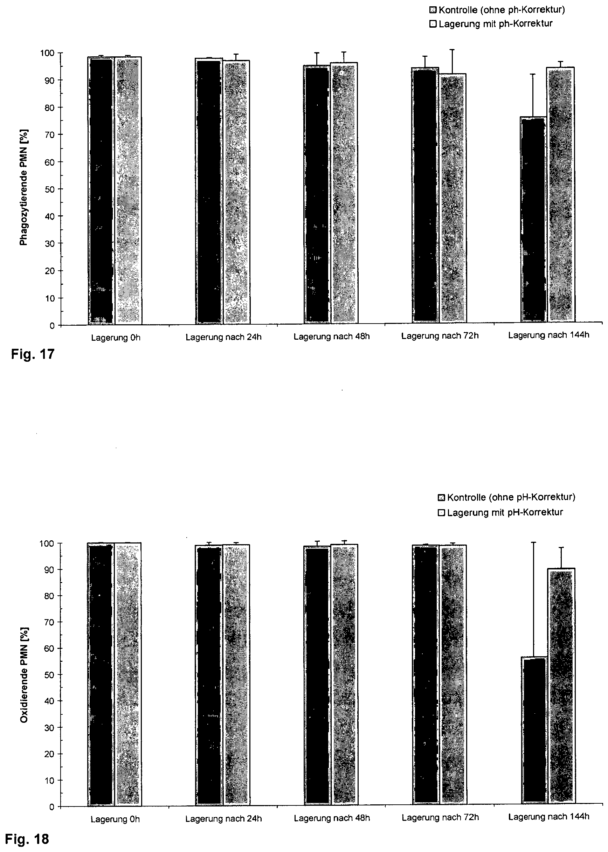

Fig. 17 zeigt, dass sich die relative Anzahl phagozytierender Granulozyten (PMN, "polymorph nuclear leucocytes") bis 72 Stunden nach Lagerungsbeginn bei beiden Lagerungsvarianten kaum verändert und >90% ist. Es wird aber deutlich sichtbar, dass die relative Anzahl der phagozytierenden Granulozyten, die in einem gepufferten CPD-Plasma gelagert wurden, auch nach 144 Stunden Lagerungsdauer im Gegensatz zum ungepufferten Kontrollansatz konstant blieb.

In Fig. 18 ist der prozentuale Anteil an Granulozyten gezeigt, die Sauerstoffradikale produzieren ("oxidative burst"), in der Legende vereinfacht als "oxidierende PMN" beschrieben. Dieser beträgt sowohl beim Kontrollansatz als auch bei der Lagerung mit pH-Korrektur bis 72 Stunden annähernd 100%. Auch hier wird der positive Einfluss der pH-Wert-Korrektur auf die relative Anzahl oxidierender Granulozyten nach 144 Stunden sichtbar, wobei in der Kontrolle eine hohe Schwankungsbreite zu sehen ist, die z.T. auf Spenderunterschiede zurückzuführen sein dürfte.

Mit der im vierten Ausführungsbeispiel beschriebenen Lagerungsvariante (Lagerung mit pH-Korrektur) und den gezeigten Ergebnissen konnte gezeigt werden, dass eine Lagerung von Granulozyten ohne wesentliche Beeinträchtigung von Zellzahl, Vitalität und relativer Anzahl an phagozytierender und oxidierender Zellen über einen Zeitraum von 144 Stunden nach Aufreinigung möglich ist.

### Fünftes Ausführungsbeispiel

Das fünfte Ausführungsbeispiel beschreibt die Herstellung einer Leukozytenpräparation aus Granulozytenapheresaten nach der im dritten Ausführungsbeispiel beschriebenen Variante 2 und der anschließenden Lagerung in blutgruppenidentischem CPD-Plasma mit der Additivlösung PAGGS-M über einen Zeitraum von 72 Stunden.

Die Additivlösung PAGGS-M enthält neben Natriumchlorid, Natriumdihydrogenphosphat-Dihydrat und Dinatriumhydrogenphosphat-Dihydrat auch Glukose-Monohydrat, Mannitol, Adenin und Guanosin.

Im Anschluss an die Sedimentation und Aufreinigung des Granulozytenapheresats wurde die Zellkonzentration der beiden Lagerungsansätze auf 5•10⁷ Leukozyten/ml eingestellt, so dass 1•10¹⁰ Leukozyten in 200 ml Lagerungsmedium in einem Thrombozytenlagerungsbeutel ohne Agitation bei Raumtemperatur gelagert wurden. Ein Versuchsansatz enthielt als Lagerungsmedium reines blutgruppenidentisches CPD-Plasma (Kontrolle). Der andere wurde mit einem Gemisch aus CPD-Plasma mit einem Volumenanteil von 35% der Additivlösung PAGGS-M versetzt. Die Thrombozytenlagerungsbeutel lagen während der gesamten Lagerungsdauer auf Gitterrosten, um vollen Kontakt der Beuteloberfläche mit der Luft zu gewährleisten. Bei beiden Lagerungsansätzen wurde der pH-Wert mit TRIS 36,34% bei einem pH-Wert von kleiner als 7,2 so korrigiert, dass er sich wieder im physiologischen Bereich von humanem Blut (7,35 - 7,45) befand.

### Analyse

Nach 0, 24, 48 und 72 Stunden wurden die beiden Leukozytenlagerungsansätze gut gemischt und jeweils Proben von 3 ml zur Analyse unter sterilen Bedingungen entnommen.

Zu jedem Zeitpunkt wurden die Zellkonzentration und die Gesamtzellzahl der Proben bestimmt. Weiterhin wurde die Vitalität mittels Trypanblau-Ausschlusstest ermittelt und die Funktionalität im Bereich Phagozytose und respiratorischer Burst mit der Fluoreszenzzytometrie untersucht. Die Durchführung aller Analysen erfolgte wie im ersten Ausführungsbeispiel detailliert beschrieben.

### Resultate

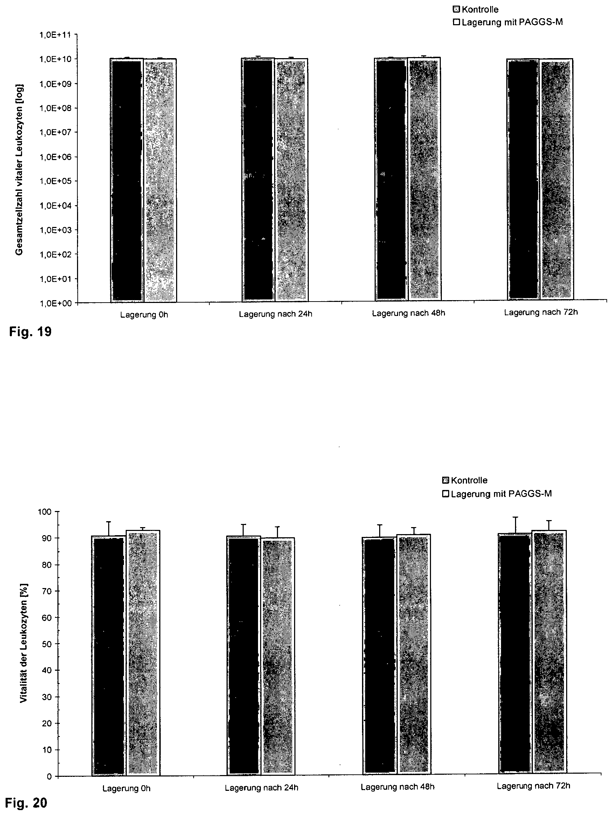

Die in den Figuren 19 bis 24 dargestellten Daten sind Mittelwerte und deren Standardabweichungen.

Anhand von Fig. 19 und 20 ist festzustellen, dass sowohl die Gesamtzellzahl an vitalen Leukozyten als auch die Vitalität (>90%) über die Lagerung von 72 Stunden stabil bleibt. So kann kein negativer Einfluss durch die Additivlösung erkannt werden.

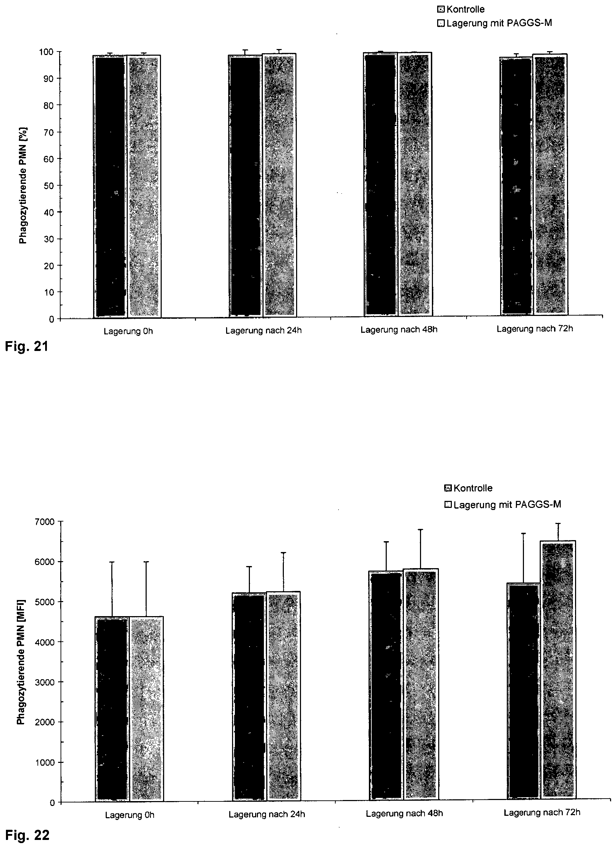

Aus Fig. 21 ist erkennbar, dass die relative Anzahl phagozytierender Granulozyten (PMN, "polymorph nuclear leucocytes") bis 72 Stunden nach Lagerungsbeginn bei beiden Lagerungsvarianten sich kaum verändert und bei fast 100% liegt. In Fig. 22 ist die durchschnittliche Fluoreszenz-Intensität (MFI, "mean fluorescence index") pro einzelne Zelle, bezogen auf die Ergebnisse aus Fig. 21, dargestellt. Daraus geht hervor, dass es durch die Zugabe der Additivlösung PAGGS-M zu einer Steigerung der Phagozytose-Leistung pro Zelle über 72 Stunden kommt, wohingegen der Kontrollansatz über den gleichen Zeitraum gleichbleibend stark bleibt.

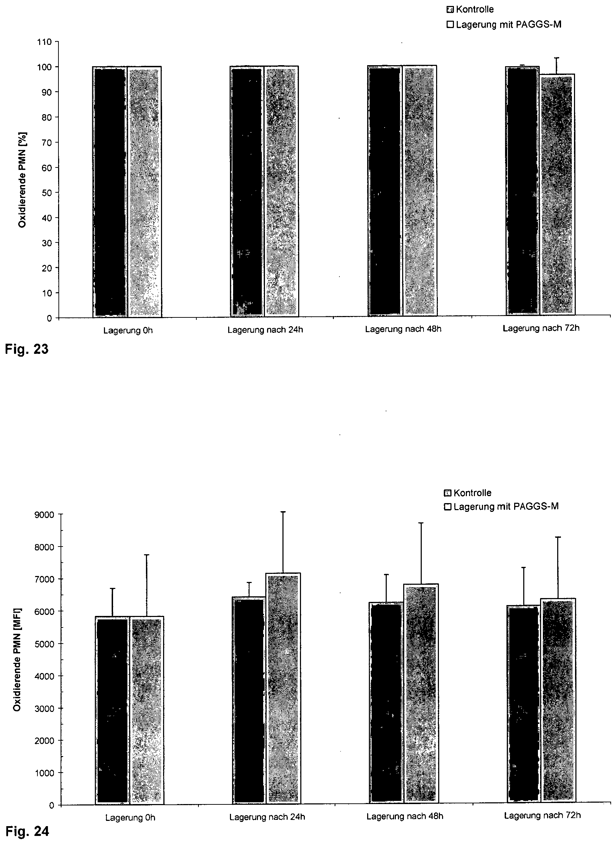

In Fig. 23 ist die relative Anzahl von oxidierenden Granulozyten gezeigt. Diese beträgt bei beiden Lagerungsansätzen über 72 Stunden annähernd 100%. Der in Fig. 24 dargestellte MFI pro oxidierende Zelle lässt darauf schließen, dass die Granulozyten in ihrer Aktivität in der Kontrolle über den Lagerungszeitraum von 72 Stunden gleichbleibend stark bleiben. Bei Lagerung mit Additivlösung ist die Aktivität der Granulozyten etwas erhöht.

Durch die im fünften Ausführungsbeispiel beschriebene Lagerungsmethode (Lagerung mit Additivlösung PAGGS-M) konnte nicht nur gezeigt werden, dass eine Lagerung von Granulozyten ohne wesentliche Beeinträchtigung von Zellzahl, Vitalität, relativer Anzahl an phagozytierender und oxidierender Zellen und deren Leistung über einen Zeitraum von 72 Stunden nach Aufreinigung möglich ist, sondern das die Zugabe von PAGGS-M die Leistungsfähigkeit der Granulozyten verbessern kann.

### Sechstes Ausführungsbeispiel

Das sechste Ausführungsbeispiel beschreibt die Herstellung einer Leukozytenpräparation aus Granulozytenapheresaten nach der im dritten Ausführungsbeispiel beschriebenen Variante 2 und der anschließenden Lagerung in blutgruppenidentischem CPD-Plasma mit 35% PAGGS-M und dem Immunpräparat "Immuno-akut®" der Firma MensSana AG über einen Zeitraum von 120 Stunden.

Das Immunpräparat "Immuno-akut®" enthält neben den Vitaminen B6, B12, C, D und E, auch Folsäure, diverse Carotinoide und Spurenelemente.

Im Anschluss an die Sedimentation und Aufreinigung des Granulozytenapheresats wurde die Zellkonzentration der beiden Lagerungsansätze auf 5•10⁷ Leukozyten/ml eingestellt, so dass 1•10¹⁰ Leukozyten in 200 ml CPD-Plasma-PAGGS-M-Gemisch in einem Thrombozytenlagerungsbeutel ohne Agitation bei Raumtemperatur (22 ± 2°C) gelagert wurden. Zu einem der beiden Lagerungsansätze wurden ca. 30 mg des Immunpräparat-Pulvers aufgelöst und steril filtriert zugegeben. Die Thrombozytenlagerungsbeutel lagen während der gesamten Lagerungsdauer auf Gitterrosten, um vollen Kontakt der Beuteloberfläche mit der Luft zu gewährleisten. Bei beiden Lagerungsansätzen wurde bei Bedarf der pH-Wert (bei <7,2) mit TRIS 36,34% so korrigiert, dass er sich wieder im physiologischen Bereich von humanem Blut (7,35 - 7,45) befand.

### Analyse

Nach 0, 24, 48, 72, 96 und 120 Stunden wurden die beiden Leukozytenlagerungsansätze gut gemischt und jeweils Proben von 3 ml zur Analyse unter sterilen Bedingungen entnommen.

Zu jedem Zeitpunkt wurden die Zellkonzentration und die Gesamtzellzahl der Proben bestimmt. Weiterhin wurde die Vitalität mittels Trypanblau-Ausschlusstest ermittelt und die Funktionalität im Bereich Phagozytose und oxidativer Burst mit der Fluoreszenzzytometrie untersucht. Der respirative Burst wurde zusätzlich noch mittels Chemilumineszenz-Messung mit Lucigenin gemessen. Die Durchführung aller Analysen erfolgte wie im ersten Ausführungsbeispiel detailliert beschrieben.

### Resultate

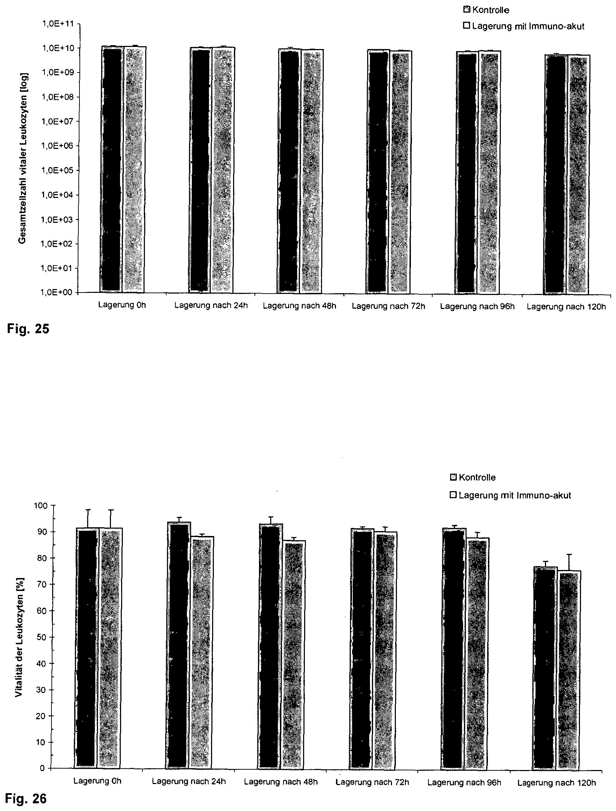

Die in den Figuren 25 bis 31 dargestellten Daten sind Mittelwerte und deren Standardabweichungen.

Anhand von Fig. 25 ist festzustellen, dass die Gesamtzellzahl an vitalen Leukozyten über den Lagerungszeitraum von 120 Stunden stabil bleibt. Durch Fig. 26 wird deutlich, dass die Vitalität bei beiden Lagerungsvarianten über 96 Stunden konstant bleibt, und erst nach 120 Stunden sich minimal verschlechtert. Während der Lagerungsdauer wird aber kein negativer Einfluss durch das Immuno-akut® deutlich.

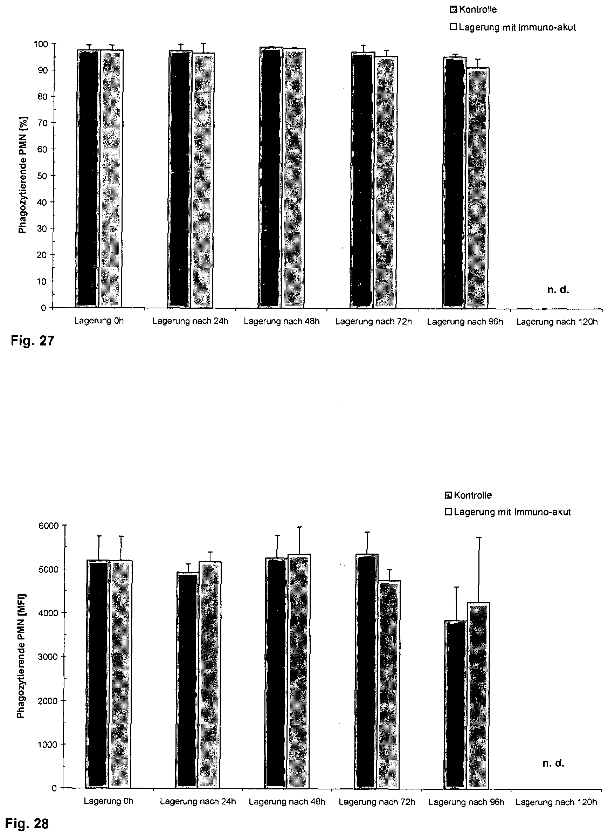

Aus Fig. 27 ist erkennbar, dass sich die relative Anzahl phagozytierender Granulozyten (PMN, "polymorph nuclear leucocytes") über 96 Stunden der Lagerung bei beiden Lagerungsvarianten kaum verändert und bei fast 100% liegt. In Fig. 28 ist die durchschnittliche Fluoreszenz-Intensität (MFI, "mean fluorescence index") pro einzelne Zelle dargestellt. Es zeigt sich, dass beide Lagerungsansätze eine gleichbleibend gute Phagozytose-Leistung über 72 Stunden haben. Lediglich nach 96 Stunden verschlechtert sich die Leistung beider Ansätze minimal, wobei sie bei der Lagerung mit dem Immunpräparat etwas geringer ausfällt.

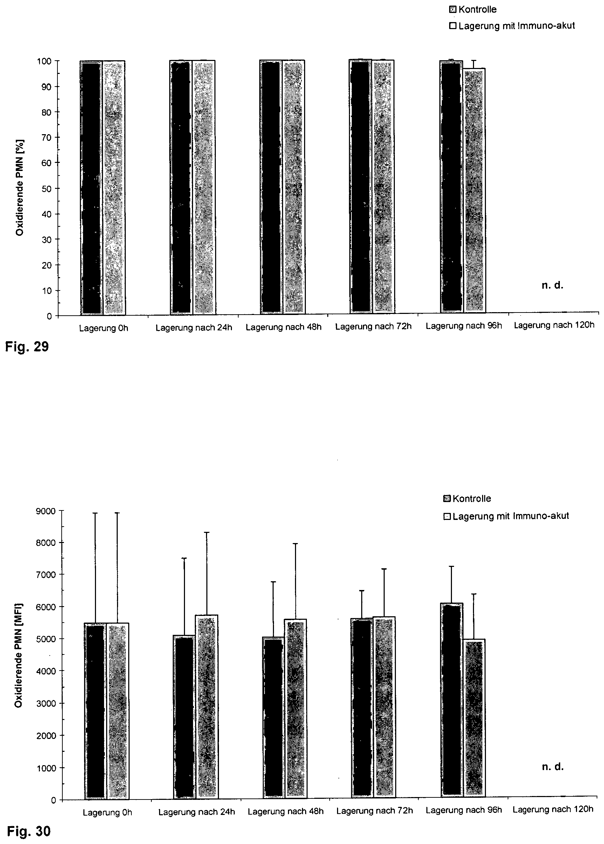

Ein ähnliches Bild zeigt sich auch für den mit Fluoreszenzdurchflusszytometrie gemessenen oxidativen Burst. In Fig. 29 ist die relative Anzahl von oxidierenden Granulozyten gezeigt. Diese beträgt bei beiden Lagerungsansätzen über 96 Stunden annähernd 100%. Der in Fig. 30 dargestellte MFI pro oxidierende Zelle bleibt ebenfalls über 96 Stunden bei beiden Lagerungen konstant gut.

In Fig. 31 ist das Ergebnis der Messung des respirativen Bursts der beiden Lagerungsansätze durch Messung der Chemilumineszenz mit Lucigenin nach Stimulation der PMN mit Zymosan dargestellt. Der oxidative Burst blieb über 72 Stunden bei beiden Lagerungen konstant hoch. Nach 96 bzw. 120 Stunden zeigt sich eine minimale Verschlechterung des Oxyburst.

Durch den im sechsten Ausführungsbeispiel beschriebenen Lagerungszusatz (Immunpräparat "Immuno-akut®") konnte gezeigt werden, dass eine Lagerung von Granulozyten ohne wesentliche Beeinträchtigung von Zellzahl, Vitalität, relativer Anzahl an phagozytierenden und oxidierenden Zellen und deren Leistung über einen Zeitraum von 96 Stunden nach Aufreinigung möglich ist.

### Siebtes Ausführungsbeispiel

Das siebte Ausführungsbeispiel beschreibt die teilweise maschinelle/teilautomatisierte Herstellung einer Leukozytenpräparation aus einem Granulozytenapheresat bzw. einem Leukozytenfilm-Pool. Die Analysen erfolgten analog zum ersten Ausführungsbeispiel.

Die einzelnen Leukozytenfilme wurden analog zum ersten Ausführungsbeispiel gepoolt. Die Herstellung der Granulozytenapheresate erfolgte analog zum zweiten Ausführungsbeispiel.

Unter sterilen Bedingungen wurde das Granulozytenkonzentrat bzw. der Leukozytenfilmpool direkt im Spenden- bzw. Poolbeutel mit HES 6% (MW: 200.000) im Verhältnis 1:0,5 gemischt. Anschließend wurde ein Pumpenschlauch steril mit dem Spenden-/Poolbeutel konnektiert. Das andere Schlauchende wurde ebenfalls steril mit einem Leerbeutel (Sammelbeutel) verbunden. Der Spenden- bzw. Pool-beutel wurde mit dem Pumpenschlauch nach oben zeigend zur Sedimentation aufgehängt. Während der Sedimentationsphase (30 bis 40 Minuten bei Raumtemperatur) wurde der Beutel in regelmäßigen Abständen auf eine scharfe Trennschicht zwischen dem Erythrozytenpellet und leukozytenreichen Überstand visuell kontrolliert. Sobald eine solche Trennschicht vorhanden war, wurde der leukozytenhaltige Überstand durch Anlegen eines Differenzdruckes nach oben aus dem Spenden- bzw. Poolbeutel über den Schlauch in den Sammelbeutel gepumpt und der Spenden-/Poolbeutel steril abgeschweißt. Je nach Volumen des Präparates wurde die Flussrate so gewählt, dass die Sedimentationszeit nicht wesentlich beeinflusst wird (20 bis 70 ml/min).

Nach dem Abpumpen wurde ein weiterer Leerbeutel (Abfallbeutel) an den Sammelbeutel steril angeschweißt. Der Sammelbeutel (leukozytenreicher Überstand) und der leere Abfallbeutel wurden zusammen bei 4°C, 300 g, 5 Minuten zentrifugiert. Nach der Zentrifugation wurde der Sammelbeutel in eine manuelle Blutpresse überführt und der Überstand in den Abfallbeutel gepresst. Der Abfallbeutel wurde dann steril dekonnektiert und verworfen. Um den Großteil der Thrombozyten zu entfernen, wurde das Leukozytenpellet im Sammelbeutel zweimal mit physiologischer NaCI-Lösung (0,9%) und Zentrifugation bei 300 g bei 4°C 5 Minuten gewaschen. Der Überstand wurde jeweils in einen Abfallbeutel abgepresst und verworfen. Im Anschluss an den zweiten Waschschritt wurde das aufgereinigte Granulozytenkonzentrat bzw. der gereinigte Leukozytenfilmpool in blutgruppenidentischem CPD-Plasma aufgenommen und analysiert.

Durch die hier beschriebene teils automatisierte Variante der Aufreinigung von Granulozytenapheresaten bzw. Leukozytenfilmpools konnte ebenfalls eine Abreicherung von >95% der Erythrozyten und >93% der Thrombozyten im Vergleich zum Granulozytenapheresat/Leukozytenpool erreicht werden. Durch diese Handhabung kommt es aber zu einem deutlich geringeren Verlust an Leukozyten. So konnten im Vergleich zu den im ersten bzw. zur Variante 2 des dritten Ausführungsbeispiels beschriebenen manuellen Methoden und deren Ergebnissen circa 20% der Leukozyten mehr gewonnen werden.

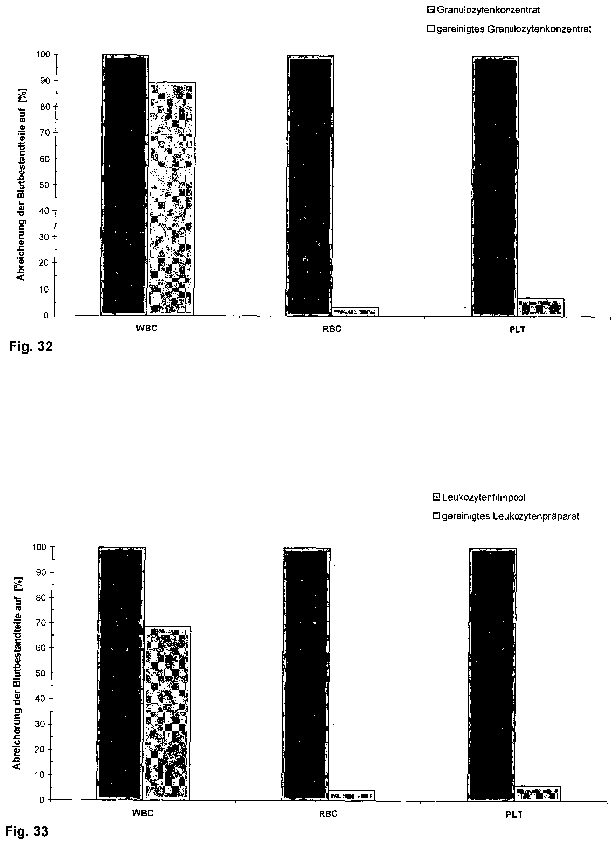

In Fig. 32 ist die Abreicherung der einzelnen Blutbestandteile Leukozyten (WBC, "white blood cells"), Erythrozyten (RBC, "red blood cells") und Thrombozyten (PLT, "platelets") für die Aufreinigung eines Granulozytenapheresats dargestellt. So wird ersichtlich, dass durch die teils automatisierte Aufreinigung 89,6% der Leukozyten (manuelle Methode: 70% Leukozytengehalt) zurückgewonnen werden konnten, obwohl die Abreicherung von Erythrozyten (96%) und der Thrombozyten (93%) im Vergleich zur manuellen Methode gleich gut ausfiel.

Fig. 33 zeigt die Abreicherungsergebnisse für ein Leukozytenpräparat, das aus einem Leukozytenfilmpool hergestellt wurde. Auch hier konnte die Ausbeute an Leukozyten (68,6%; manuelle Methode: 49,8%) bei gleichbleibend sehr guter Abreicherung an Erythrozyten (96%) und Thrombozyten (94%) verbessert werden.

Durch weitere Automatisierung auch der Waschschritte (Druckdifferenz bzw. Pumpen) ließ sich die Ausbeute der Leukozyten noch weiter steigern (ohne Abbildung).

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

## Patentansprüche

1. Verfahren zum Herstellen einer Leukozytenpräparation aus einer Leukozytenfraktion mit den folgenden Verfahrensschritten:
a) Sedimentieren der Leukozytenfraktion, bis sich eine Trennlinie zwischen den sedimentierten Erythrozyten und einem Leukozyten-Überstand gebildet hat, Abtrennen des Leukozyten-Überstands vom Sediment, wobei der Leukozyten-Überstand in einem Leukozyten-Behälter aufgefangen wird oder verbleibt,
b) Sedimentieren der Leukozyten im Leukozyten-Behälter, wobei sich ein leukozytenarmer Überstand bildet, und Entfernen des leukozytenarmen Überstands aus dem Leukozyten-Behälter,
c) Waschen des Sediments im Leukozyten-Behälter mit einer Kochsalz enthaltenden Lösung, wobei die sedimentierten Zellen in der Kochsalz enthaltenden Lösung resuspendiert werden, die Lösung sedimentiert und ein resultierender Überstand entfernt wird, und
d) Resuspendieren des Sediments im Leukozyten-Behälter in einer Lagerlösung,
e) Lagern der Lagerlösung mit dem resuspendierten Sediment in einem gasdurchlässigen Beutel.

2. Verfahren nach Anspruch 1, **dadurch gekennzeichnet, dass** die Lagerung der Lösung im Verfahrensschritt e) bei einer Temperatur von -200°C bis +40°C, insbesondere bei +2°C bis +38°C, insbesondere bei +20°C bis +25°C erfolgt, oder in Form einer Kryokonservierung bei -50°C bis -200°C.

3. Verfahren nach Anspruch 1 oder 2, **dadurch gekennzeichnet, dass** in Verfahrensschritt a) das Sedimentieren bei einer Beschleunigung von 0,5 g bis 1000 g, insbesondere von 0,5 g bis 1,5 g oder von 50 g bis 1000 g, und/oder unter Zusatz eines Sedimentationsbeschleuniger, insbesondere Hydroxyethylstärke, Dextran, Ficoll, Percoll und/oder Gelatine, erfolgt, wobei insbesondere die Sedimentationsdauer zwischen 5 und 120 Minuten beträgt, vorzugsweise zwischen 20 und 45 Minuten, und/oder das Sedimentieren der Leukozyten in Verfahrensschritt b) und/oder das Sedimentieren in Verfahrensschritt c) durch Zentrifugieren erfolgt, insbesondere bei einer Beschleunigung von 50 bis 10000 g, insbesondere bei 100 bis 5500 g, insbesondere bei 200 bis 500 g.

4. Verfahren nach einem der Ansprüche 1 bis 3, **dadurch gekennzeichnet, dass** die Waschung oder Waschungen in Verfahrensschritt c) ein oder mehrere Male wiederholt wird und/oder mittels Zentrifugieren und/oder Filtration und/oder mit einer physiologischen Lösung erfolgt oder erfolgen, die insbesondere 50 bis 200 mM Natriumchlorid und/oder weitere Salze und/oder Puffer enthält.

5. Verfahren nach einem der Ansprüche 1 bis 6, **dadurch gekennzeichnet, dass** die Lagerlösung ein blutgruppengleiches Blutplasma und/oder eine wässrige Lösung ist, die insbesondere blutgruppengleiches Plasma enthält und/oder dass vor, während und/oder nach einem der Verfahrensschritte a) bis e) mehrere Präparate gepoolt werden.

6. Verfahren nach einem der Ansprüche 1 bis 5, **dadurch gekennzeichnet, dass** das Verfahren in einem geschlossenen System steril erfolgt, insbesondere in einem System von Schläuchen und Beuteln.

7. Verfahren nach einem der Ansprüche 1 bis 6, **dadurch gekennzeichnet, dass** vor, während oder nach einem der Verfahrensschritte a) bis d) zur Verbesserung der Lagerung Additive zugegeben werden, insbesondere aus den Gruppen der pH-stabilisierenden Substanzen, insbesondere Puffer, der Nährstoffe, insbesondere Glukose, der Funktionsverbesserer, insbesondere Zytokine und/oder Wachstumsfaktoren, der Blutplasma-Komponenten, insbesondere blutgruppengleiches Blutplasma und/oder Albumin, und/oder der Antioxidantien, insbesondere Ascorbinsäure, Glutathion, Cystein, oder Methionin.

8. Verfahren nach einem der Ansprüche 1 bis 7, **dadurch gekennzeichnet, dass** vor oder nach wenigstens einem der Verfahrensschritte a) bis e) oder unmittelbar vor Anwendung eine Bestrahlung, insbesondere mit γ-Strahlen, stattfindet, insbesondere mit einer Dosis von wenigstens 25 Gy, insbesondere wenigstens 30 Gy.

9. Verfahren nach einem der Ansprüche 1 bis 8, **dadurch gekennzeichnet, dass** die Überführung eines Anteils aus einem Beutel in einen anderen durch Druckdifferenz erfolgt, die manuell oder maschinell oder unter Ausnutzung der Schwerkraft erzeugt wird.

10. Leukozytenpräparation, hergestellt oder herstellbar in einem Verfahren nach einem der Ansprüche 1 bis 9, wobei gegenüber Vollblut die Erythrozyten um mehr als 90%, insbesondere mehr als 98%, und die Thrombozyten um mehr als 90% reduziert sind, während die Leukozyten um nicht mehr als 60% reduziert sind, wobei sie bei einer Temperatur zwischen 2 °C und 38 °C für eine Dauer von mehr als 24 Stunden bis 168 Stunden ohne wesentlichen Funktionsverlust lagerbar ist, wobei insbesondere der respiratorische Burst und/oder die Phagozytose auch nach 72 Stunden der Lagerung noch mehr als 90% der entsprechenden Werte der frischen Leukozytenpräparation beträgt oder betragen.

11. Leukozytenpräparation nach Anspruch 10, geeignet zur Verwendung zur Herstellung eines im Wesentlichen zellfreien Überstandes mit hoher Mediatorkonzentration und/oder zur Verwendung in einem extrakorporalen Kreislauf oder zur Infusion.

## Claims

1. Method for preparing a leukocyte preparation from a leukocyte fraction, comprising the following method steps:
a) sedimenting the leukocyte fraction until a dividing line has formed between the sedimented erythrocytes and a leukocyte supernatant, removing the leukocyte supernatant from the sediment, wherein the leukocyte supernatant is collected or remains in a leukocyte container,
b) sedimenting the leukocytes in the leukocyte container, wherein a low-leukocyte supernatant is formed, and removing the low-leukocyte supernatant from the leukocyte container,
c) washing the sediment in the leukocyte container with a saline solution, wherein the sedimented cells are resuspended in the saline solution, the solution is sedimented and a resulting supernatant is removed, and
d) resuspending the sediment in the leukocyte container in a storage solution,
e) storing the storage solution in a gas-permeable bag.

2. Method according to Claim 1, **characterized in that** in method step e) the solution is stored at a temperature from -200°C to +40°C, more particularly at +2°C to +38°C, more particularly at +20°C to +25°C, or at -50°C to -200°C in a cryopreservation procedure.

3. Method according to Claim 1 or 2, **characterized in that** sedimentation is carried out in method step a) at an acceleration from 0.5 g to 1000 g, more particularly from 0.5 g to 1.5 g or from 50 g to 1000 g, and/or with the addition of a sedimentation accelerator, more particularly hydroxyethyl starch, dextran, Ficoll, Percoll and/or gelatin, wherein the sedimentation time in particular is between 5 and 120 minutes, preferably between 20 and 45 minutes, and/or the sedimentation of the leukocytes in method step b) and/or sedimentation in method step c) is carried out by centrifugation, more particularly at an acceleration from 50 to 10000 g, more particularly at 100 to 5500 g, more particularly at 200 to 500 g.

4. Method according to any of Claims 1 to 3, **characterized in that** the wash or washes in method step c) is repeated one or more times and/or is or are carried out by means of centrifugation and/or filtration and/or with a physiological solution containing in particular 50 to 200 mM sodium chloride and/or further salts and/or buffers.

5. Method according to any of Claims 1 to 4, **characterized in that** the storage solution is a blood group-identical blood plasma and/or an aqueous solution containing in particular blood group-identical plasma, and/or multiple preparations are pooled before, during and/or after any of method steps a) to e).

6. Method according to any of Claims 1 to 5, **characterized in that** the method is carried out aseptically in a closed system, more particularly in a system of tubing and bags.

7. Method according to any of Claims 1 to 6, **characterized in that**, before, during or after any of method steps a) to d), additives are added, more particularly from the groups of the pH-stabilizing substances, more particularly buffer, of the nutrients, more particularly glucose, of the function improvers, more particularly cytokines and/or growth factors, of the blood plasma components, more particularly blood group-identical blood plasma and/or albumin, and/or of the antioxidants, more particularly ascorbic acid, glutathione, cysteine, or methionine, in order to improve storage.

8. Method according to any of Claims 1 to 10, **characterized in that**, before or after at least one of method steps a) to e) or immediately before use, an irradiation, more particularly with γ-rays, takes place, more particularly with a dose of at least 25 Gy, more particularly at least 30 Gy.

9. Method according to any of Claims 1 to 8, **characterized in that** a portion is transferred from one bag to another by a pressure difference generated manually or by machine or by utilizing gravity.

10. Leukocyte preparation prepared or obtainable in a method according to any of Claims 1 to 9, wherein, with respect to whole blood, the erythrocytes are reduced by more than 90%, more particularly more than 98%, and the thrombocytes by more than 90%, whereas the leukocytes are reduced by not more than 60%, wherein it is storable at a temperature between 2°C and 38°C for a period of more than 24 hours to 168 hours without substantial loss of function, wherein in particular respiratory burst and/or phagocytosis is or are still, even after 72 hours of storage, more than 90% of the corresponding values of the fresh leukocyte preparation.

11. Leukocyte preparation according to claim 10, suitable for preparing a substantially cell-free supernatant having a high mediator concentration and/or for use in an extracorporeal circulation or for infusion.

## Revendications

1. Procédé permettant de fabriquer une préparation de leucocytes à partir d'une fraction de leucocytes, comprenant les étapes de procédé suivantes :
a) sédimenter la fraction de leucocytes jusqu'à ce qu'une ligne de séparation se forme entre les érythrocytes sédimentés et un surnageant de leucocytes, éliminer le surnageant de leucocytes du sédiment, le surnageant de leucocytes étant collecté ou restant dans un récipient de leucocytes,
b) sédimenter les leucocytes dans le récipient de leucocytes, de sorte qu'un surnageant pauvre en leucocytes soit formé, et éliminer le surnageant de leucocytes du récipient de leucocytes,
c) laver le sédiment dans le récipient de leucocytes avec une solution saline, les cellules sédimentées étant remises en suspension dans la solution saline, la solution est sédimentée et un surnageant qui en résulte étant éliminé, et
d) remettre le sédiment présent dans le récipient de leucocytes en suspension dans une solution de stockage,
e) stocker dans un sac perméable aux gaz la solution de stockage avec le sédiment remis en suspension.

2. Procédé selon la revendication 1, **caractérisé en ce que** le stockage de la solution dans l'étape de procédé e) est effectué à une température allant de -200°C à +40°C, en particulier de +2°C à +38°C, en particulier de +20°C à +25°C, ou sous la forme d'une cryoconservation entre -50°C et -200°C.

3. Procédé selon la revendication 1 ou la revendication 2, **caractérisé en ce que**, dans l'étape a) du procédé, la sédimentation s'effectue selon une accélération allant de 0,5 g à 1000 g, en particulier de 0,5 g à 1,5 g ou de 50 g à 1000 g, et / ou par l'ajout d'un accélérateur de sédimentation, en particulier de l'hydroxyéthylamidon, du dextrane, du ficoll, du percoll et / ou de la gélatine, le temps de sédimentation étant en particulier compris entre 5 et 120 minutes, de préférence entre 20 et 45 minutes, et / ou la sédimentation des leucocytes dans l'étape de procédé b) et / ou la sédimentation dans l'étape de procédé c) est effectuée par centrifugation, en particulier selon une accélération allant de 50 à 10000 g, en particulier de 100 à 5500 g, en particulier de 200 à 500 g.

4. Procédé selon l'une des revendications 1 à 3, **caractérisé en ce que** le lavage ou les lavages sont répétés une ou plusieurs fois dans l'étape c) du procédé et / ou se déroule ou se déroulent par centrifugation et / ou filtration et / ou avec l'ajout d'une solution physiologique, en particulier du chlorure de sodium à 50 à 200 mM et / ou d'autres sels et / ou tampons.

5. Procédé l'une des revendications 1 à 4, **caractérisé en ce que** la solution de stockage est un plasma sanguin de groupe sanguin identique et / ou une solution aqueuse, notamment contenant du plasma de groupe sanguin identique et / ou plusieurs préparations sont regroupées avant, pendant et / ou après l'une des étapes du procédé a) à e).

6. Procédé selon l'une des revendications 1 à 5, **caractérisé en ce que** le procédé est mis en oeuvre de façon stérile dans un système fermé, en particulier dans un système de tubes et de sacs.

7. Procédé selon l'une des revendications 1 à 6, **caractérisé en ce que**, avant, pendant ou après l'une des étapes de procédé a) à d), des additifs sont ajoutés pour l'amélioration du stockage, en particulier choisis dans le groupe comprenant des substances stabilisatrices du pH, en particulier des tampons, des nutriments, en particulier du glucose, des améliorateurs fonctionnels, en particulier des cytokines et / ou des facteurs de croissance, des composants du plasma sanguin, en particulier du plasma sanguin de groupe sanguin identique et / ou de l'albumine, et / ou des antioxydants, en particulier de l'acide ascorbique, du glutathion, de la cystéine ou de la méthionine.

8. Procédé selon l'une des revendications 1 à 7, **caractérisé en ce que**, **caractérisé en ce que**, avant ou après au moins une des étapes de procédé a) à e) ou immédiatement avant l'utilisation, une irradiation est faite, en particulier avec des rayons γ, notamment avec une dose d'au moins 25 Gy, en particulier au moins 30 Gy.

9. Procédé selon l'une des revendications 1 à 8, **caractérisé en ce qu'**un transfert d'une partie de la préparation d'une poche à l'autre s'effectue par différence de pression qui est générée manuellement ou par machine ou en utilisant la gravité.

10. Préparation de leucocytes, produite ou apte à être produite selon un procédé selon l'une des revendications 1 à 9, dans laquelle, par rapport au sang complet, les érythrocytes sont réduits de plus de 90%, en particulier de plus de 98%, et les thrombocytes sont réduits de plus de 90%, tandis que les leucocytes ne sont pas réduits de plus de 60% et peuvent être stockés à une température comprise entre 2°C et 38°C pendant une période de plus de 24 heures à 168 heures sans perte substantielle de fonction, en particulier la salve respiratoire et / ou la phagocytose est/sont ou représente(nt) toujours plus de 90% des valeurs correspondantes de la préparation de leucocytes frais, même après 72 heures de stockage.

11. Préparation de leucocytes selon la revendication 10, apte à être utilisée pour la fabrication d'un surnageant essentiellement exempt de cellules ayant une forte concentration de médiateur et / ou pour une utilisation dans une circulation extracorporelle ou pour une perfusion.
